# EUROPEAN PATENT SPECIFICATION

(11) **EP 2 839 010 B1**
(45) Date of publication and mention of the grant of the patent: **25.04.2018**
(21) Application number: 13765199.8
(22) Date of filing: 19.03.2013
(51) Int. Cl.: C12P 21/06, C12N 15/62, C07K 14/575

(54) **METHOD OF PRODUCING A RECOMBINANT PEPTIDE**
VERFAHREN ZUR HERSTELLUNG EINES REKOMBINANTEN PEPTIDS
PROCÉDÉ DE PRODUCTION D'UN PEPTIDE RECOMBINANT

(30) Priority: 19.03.2012 US 201261612817 P
(43) Date of publication of application: 25.02.2015
(73) Proprietor: Madeleine Pharmaceuticals Pty Ltd, Mount Barker, SA 5251 (AU)
(72) Inventor: BASTIRAS, Stan, Adelaide, South Australia 5000 (AU); GUIDOLIN, Angelo, Parkside, South Australia 5063 (AU); HUNT, Ben, Modbury North, South Australia 5092 (AU); RAISHEED, Saif, Glenside, South Australia 5065 (AU); ZAREIE, Reza, Churchlands, Western Australia 6018 (AU)
(74) Representative: Gill Jennings & Every LLP
(86) International application number: PCT/AU2013/000273
(87) International publication number: WO 2013/138850

(56) References cited:
- WO-A1-2012/019237
- WO-A2-2011/033375
- US-A1- 2004 265 910
- US-A1- 2009 176 706
- US-B1- 6 558 924
- JONASSON P ET AL: "SINGLE-STEP TRYPSINCLEAVAGE OF A FUSION PROTEIN TO OBTAIN HUMAN INSULIN AND ITS C PEPTIDE", EUROPEAN JOURNAL OF BIOCHEMISTRY, WILEY-BLACKWELL PUBLISHING LTD, GB, vol. 236, no. 2, 1 March 1996 (1996-03-01) , pages 656-661, XP008071709, ISSN: 0014-2956, DOI: 10.1111/J.1432-1033.1996.00656.X

### SEQUENCE LISTING

<110> HOSPIRA ADELAIDE PTY LTD
<120> Method of producing a recombinant peptide
<130> 39357PCT
<160> 20
<170> PatentIn version 3.5
<210> 1
   <211> 8
   <212> PRT
   <213> Artificial Sequence
<220>
   <223> wherein Xaa is any amino acid except arginine or lysine, and xbb is arginine or lysine.
<220>
   <221> misc_feature
   <222> (1)..(3)
   <223> Xaa can be any naturally occurring amino acid
<220>
   <221> mist-feature
   <222> (5)..(5)
   <223> Xaa can be any naturally occurring amino acid
<400> 1
<210> 2
   <211> 37
   <212> PRT
   <213> Homo sapiens
<400> 2
   Glu Val Val Pro Pro Gln Val Leu Ser Glu Pro Asn Glu Glu Ala Gly
   1               5                   10                  15
   Ala Ala Leu Ser Pro Leu Pro Glu Val Pro Pro Trp Thr Gly Glu Val
               20                  25                  30
   Ser Pro Ala Gln Arg
           35
<210> 3
   <211> 73
   <212> PRT
   <213> Bos primigenius
<400> 3
   Met Lys Glu Val Lys Ser Leu Leu Leu Asp Leu Gln Leu Leu Leu Glu
   1               5                   10                  15
   Lys Val Lys Asn Pro Glu Asn Leu Lys Leu Ser Arg Met His Thr Phe
               20                  25                  30
   Asp Phe Tyr Val Pro Lys Val Asn Ala Thr Glu Leu Lys His Leu Lys
           35                  40                  45
   Ala Leu Leu Glu Glu Leu Lys Leu Leu Glu Glu Val Leu Asn Leu Ala
       50                  55                  60
   Pro Ser Lys Asn Leu Asn Val Asp Arg
   65                  70
<210> 4
   <211> 5
   <212> PRT
   <213> Artificial Sequence
<220>
   <223> Enteropeptidase (enterokinase) peptide cleavage site
<400> 4
   Asp Asp Asp Asp Lys
   1 5
<210> 5
   <211> 8
   <212> PRT
   <213> Artificial sequence
<220>
   <223> Tobacco etch virus protease peptide cleavage site. where X is any amino acid.
<220>
   <221> misc_feature
   <222> (2)..(3)
   <223> xaa can be any naturally occurring amino acid
<220>
   <221> misc_feature
   <222> (5)..(5)
   <223> xaa can be any naturally occurring amino acid. xbb is Gly or Ser.
<400> 5
<210> 6
   <211> 6
   <212> PRT
   <213> Artificial sequence
<220>
   <223> Tobacco etch virus protease peptide cleavage site. where X is any amino acid.
<220>
   <221> misc_feature
   <222> (1)..(1)
   <223> Xaa can be any naturally occurring amino acid
<220>
   <221> misc_feature
   <222> (3)..(3)
   <223> Xaa can be any naturally occurring amino acid. xbb is Gly or Ser.
<400> 6
<210> 7
   <211> 6
   <212> PRT
   <213> Artificial Sequence
<220>
   <223> Thrombin cleavage site.
<400> 7
   Leu Val Pro Arg Gly Ser
   1 5
<210> 8
   <211> 37
   <212> PRT
   <213> Pongo pygmaeus
<400> 8
   Glu Val Val Pro Pro Gln Val Leu Ser Glu Gln Asn Glu Glu Ala Gly
   1 5 10 15
   Ala Ala Leu Ser Pro Leu Pro Glu Val Pro Pro Trp Thr Gly Glu Val
   20 25 30
   Ser Pro Ala Gln Arg
   35
<210> 9
   <211> 37
   <212> PRT
   <213> Felis catus
<400> 9
   Glu Val Val Pro Pro Gln Val Leu Ser Glu Gln Asn Glu Glu Ala Gly
   1 5 10 15
   Ala Ala Leu Ser Pro Leu Pro Glu Val Pro Pro Trp Ala Gly Glu Val
   20 25 30
   Asn Pro Ala Gln Arg
   35
<210> 10
   <211> 73
   <212> PRT
   <213> Artificial Sequence
<220>
   <223>
<400> 10
   Met Lys Glu Val Lys Ser Leu Leu Leu Asp Leu Gln Leu Leu Leu Glu
   1 5 10 15
   Lys Val Lys Asn Pro Glu Asn Leu Lys Leu Ser Arg Met His Thr Phe
   20 25 30
   Asp Phe Tyr Val Pro Lys Val Asn Ala Thr Glu Leu Lys His Leu Lys
   35 40 45
   Ala Leu Leu Glu Glu Leu Lys Leu Leu Glu Glu Val Leu Asn Leu Ala
   50 55 60
   Pro Ser Lys Asn Leu Asn Val Gln Arg
   65 70
<210> 11
   <211> 110
   <212> PRT
   <213> Artificial Sequence
<220>
   <223>
<400> 11
   Met Lys Glu Val Lys Ser Leu Leu Leu Asp Leu Gln Leu Leu Leu Glu
   1 5 10 15
   Lys Val Lys Asn Pro Glu Asn Leu Lys Leu Ser Arg Met His Thr Phe
   20 25 30
   Asp Phe Tyr Val Pro Lys Val Asn Ala Thr Glu Leu Lys His Leu Lys
   35 40 45
   Ala Leu Leu Glu Glu Leu Lys Leu Leu Glu Glu Val Leu Asn Leu Ala
   50 55 60
   Pro Ser Lys Asn Leu Asn Val Asp Arg Glu Val Val Pro Pro Gln Val
   65 70 75 80
   Leu Ser Glu Pro Asn Glu Glu Ala Gly Ala Ala Leu Ser Pro Leu Pro
   85 90 95
   Glu Val Pro Pro Trp Thr Gly Glu Val Ser Pro Ala Gln Arg
   100 105 110
<210> 12
   <211> 184
   <212> PRT
   <213> Artificial Sequence
<220>
   <223>
<400> 12
   Met Lys Glu Val Lys Ser Leu Leu Leu Asp Leu Gln Leu Leu Leu Glu
   1               5                   10                  15
   Lys Val Lys Asn Pro Glu Asn Leu Lys Leu Ser Arg Met His Thr Phe
               20                  25                  30
   Asp Phe Tyr Val Pro Lys Val Asn Ala Thr Glu Leu Lys His Leu Lys
           35                  40                  45
   Ala Leu Leu Glu Glu Leu Lys Leu Leu Glu Glu Val Leu Asn Leu Ala
       50                  55                  60
   Pro Ser Lys Asn Leu Asn Val Asp Arg Glu Val Val Pro Pro Gln Val
   65                  70                  75                  80
   Leu Ser Glu Pro Asn Glu Glu Ala Gly Ala Ala Leu Ser Pro Leu Pro
                   85                  90                  95
   Glu Val Pro Pro Trp Thr Gly Glu Val Ser Pro Ala Gln Arg Glu Val
               100                 105                 110
   Val Pro Pro Gln Val Leu Ser Glu Pro Asn Glu Glu Ala Gly Ala Ala
           115                 120                 125
   Leu Ser Pro Leu Pro Glu Val Pro Pro Trp Thr Gly Glu Val Ser Pro
       130                 135                 140
   Ala Gln Arg Glu Val Val Pro Pro Gln Val Leu Ser Glu Pro Asn Glu
   145                 150                 155                 160
   Glu Ala Gly Ala Ala Leu Ser Pro Leu Pro Glu Val Pro Pro Trp Thr
                   165                 170                 175
   Gly Glu Val Ser Pro Ala Gln Arg
               180
<210> 13
   <211> 443
   <212> PRT
   <213> Artificial sequence
<220>
   <223>
<400> 13
   Met Lys Glu Val Lys Ser Leu Leu Leu Asp Leu Gln Leu Leu Leu Glu
   1               5                   10                  15
   Lys Val Lys Asn Pro Glu Asn Leu Lys Leu Ser Arg Met His Thr Phe
   20 25 30
   Asp Phe Tyr Val Pro Lys Val Asn Ala Thr Glu Leu Lys His Leu Lys
   35 40 45
   Ala Leu Leu Glu Glu Leu Lys Leu Leu Glu Glu Val Leu Asn Leu Ala
   50 55 60
   Pro Ser Lys Asn Leu Asn Val Asp Arg Glu Val Val Pro Pro Gln Val
   65 70 75 80
   Leu Ser Glu Pro Asn Glu Glu Ala Gly Ala Ala Leu Ser Pro Leu Pro
   85 90 95
   Glu Val Pro Pro Trp Thr Gly Glu Val Ser Pro Ala Gln Arg Glu Val
   100 105 110
   Val Pro Pro Gln Val Leu Ser Glu Pro Asn Glu Glu Ala Gly Ala Ala
   115 120 125
   Leu Ser Pro Leu Pro Glu Val Pro Pro Trp Thr Gly Glu Val Ser Pro
   130 135 140
   Ala Gln Arg Glu Val Val Pro Pro Gln Val Leu Ser Glu Pro Asn Glu
   145 150 155 160
   Glu Ala Gly Ala Ala Leu Ser Pro Leu Pro Glu Val Pro Pro Trp Thr
   165 170 175
   Gly Glu Val Ser Pro Ala Gln Arg Glu Val Val Pro Pro Gln Val Leu
   180 185 190
   Ser Glu Pro Asn Glu Glu Ala Gly Ala Ala Leu Ser Pro Leu Pro Glu
   195 200 205
   Val Pro Pro Trp Thr Gly Glu Val Ser Pro Ala Gln Arg Glu Val Val
   210 215 220
   Pro Pro Gln Val Leu Ser Glu Pro Asn Glu Glu Ala Gly Ala Ala Leu
   225 230 235 240
   Ser Pro Leu Pro Glu Val Pro Pro Trp Thr Gly Glu Val Ser Pro Ala
   245 250 255
   Gln Arg Glu Val Val Pro Pro Gln Val Leu Ser Glu Pro Asn Glu Glu
   260 265 270
   Ala Gly Ala Ala Leu Ser Pro Leu Pro Glu Val Pro Pro Trp Thr Gly
   275 280 285
   Glu Val Ser Pro Ala Gln Arg Glu Val Val Pro Pro Gln Val Leu Ser
       290                 295                 300
   Glu Pro Asn Glu Glu Ala Gly Ala Ala Leu Ser Pro Leu Pro Glu Val
   305                 310                 315                 320
   Pro Pro Trp Thr Gly Glu Val Ser Pro Ala Gln Arg Glu Val Val Pro
                   325                 330                 335
   Pro Gln Val Leu Ser Glu Pro Asn Glu Glu Ala Gly Ala Ala Leu Ser
               340                 345                 350
   Pro Leu Pro Glu Val Pro Pro Trp Thr Gly Glu Val Ser Pro Ala Gln
           355                 360                 365
   Arg Glu Val Val Pro Pro Gln Val Leu Ser Glu Pro Asn Glu Glu Ala
       370                 375                 380
   Gly Ala Ala Leu Ser Pro Leu Pro Glu Val Pro Pro Trp Thr Gly Glu
   385                 390                 395                 400
   Val Ser Pro Ala Gln Arg Glu Val Val Pro Pro Gln Val Leu Ser Glu
                   405                 410                 415
   Pro Asn Glu Glu Ala Gly Ala Ala Leu Ser Pro Leu Pro Glu Val Pro
               420                 425                 430
   Pro Trp Thr Gly Glu Val Ser Pro Ala Gln Arg
           435                 440
<210> 14
   <211> 340
   <212> DNA
   <213> Artificial Sequence
<220>
   <223> B72R-VSDL (340 bp)
<400> 14
   catatgaaag aagtgaagtc attgctgctg gatttacagt tgcttttgga gaaagttaaa      60
   aatcctgaga acctcaagct ctccaggatg catacatttg acttttacgt gcccaaggtt     120
   aacgctacag aattgaaaca tcttaaggcg ttactagaag aactcaaact tctagaggaa     180
   gtactaaatt tagctccaag caaaaacctg aacgtcgacc gtgaagttgt accgccgcag     240
   gttctgtctg aaccgaacga agaagcgggt gcggcgctgt ctccgctgcc ggaagttccg     300
   ccgtggaccg gtgaagtgag cccagcgcag cgttaagctt                           340
<210> 15
   <211> 562
   <212> DNA
   <213> Artificial Sequence
<220>
   <223> B72R-(VSDL)3 (562 bp)
<400> 15
   catatgaaag aagtgaagtc attgctgctg gatttacagt tgcttttgga gaaagttaaa      60
   aatcctgaga acctcaagct ctccaggatg catacatttg acttttacgt gcccaaggtt     120
   aacgctacag aattgaaaca tcttaaggcg ttactagaag aactcaaact tctagaggaa     180
   gtactaaatt tagctccaag caaaaacctg aacgtcgacc gtgaagttgt accgccgcag     240
   gttctgtctg aaccgaacga agaagcgggt gcggcgctgt ctccgctgcc ggaagttccg     300
   ccgtggaccg gtgaggtgag cccagcgcag cgcgaagttg taccgccgca ggttctgtct     360
   gaaccgaacg aagaagcggg tgcggcgctg tctccgctgc cggaagttcc gccatggacc     420
   ggcgaagtga gcccagcgca gcgcgaagtt gtaccgccgc aggttctgtc tgaaccgaac     480
   gaagaagcgg gtgcggcgct gtctccgctg ccggaagttc cgccatggac cggtgaagtg     540
   agcccagcgc agcgttaagc tt                                              562
<210> 16
   <211> 562
   <212> DNA
   <213> Artificial Sequence
<220>
   <223> B72(Q)R-(VSDL)3 (562 bp)
<400> 16
   catatgaaag aagtgaagtc attgctgctg gatttacagt tgcttttgga gaaagttaaa      60
   aatcctgaga acctcaagct ctccaggatg catacatttg acttttacgt gcccaaggtt     120
   aacgctacag aattgaaaca tcttaaggcg ttactagaag aactcaaact tctagaggaa     180
   gtactaaatt tagctccaag caaaaacctg aacgtccagc gtgaagttgt accgccgcag     240
   gttctgtctg aaccgaacga agaagcgggt gcggcgctgt ctccgctgcc ggaagttccg     300
   ccgtggaccg gtgaggtgag cccagcgcag cgcgaagttg taccgccgca ggttctgtct     360
   gaaccgaacg aagaagcggg tgcggcgctg tctccgctgc cggaagttcc gccatggacc     420
   ggcgaagtga gcccagcgca gcgcgaagtt gtaccgccgc aggttctgtc tgaaccgaac     480
   gaagaagcgg gtgcggcgct gtctccgctg ccggaagttc cgccatggac cggtgaagtg     540
   agcccagcgc agcgttaagc tt                                              562
<210> 17
   <211> 895
   <212> DNA
   <213> Artificial sequence
<220>
   <223> B72(Q)R-(VSDL)6 (895 bp)
<400> 17
   catatgaaag aagtgaagtc attgctgctg gatttacagt tgcttttgga gaaagttaaa      60
   aatcctgaga acctcaagct ctccaggatg catacatttg acttttacgt gcccaaggtt     120
   aacgctacag aattgaaaca tcttaaggcg ttactagaag aactcaaact tctagaggaa     180
   gtactaaatt tagctccaag caaaaacctg aacgtccagc gtgaagtggt tccgccgcag     240
   gttctgagcg aaccgaacga agaagcaggt gcagcactgt ctccgctgcc ggaagtcccg     300
   ccgtggaccg gtgaagttag tccggcgcaa cgtgaagtgg ttccgccgca ggttctgagc     360
   gaaccgaacg aagaagcagg tgcagcactg tctccgctgc cggaagtccc gccgtggacc     420
   ggtgaagtta gtccggcgca acgtgaagtg gttccgccgc aggttctgag cgaaccgaac     480
   gaagaagcag gtgcagcact gtctccgctg ccggaagtcc cgccgtggac cggtgaagtt     540
   agtccggcgc aacgtgaagt ggttccgccg caggttctga gcgaaccgaa cgaagaagca     600
   ggtgcagcac tgtctccgct gccggaagtc ccgccgtgga ccggtgaagt tagtccggcg     660
   caacgtgaag tggttccgcc gcaggttctg agcgaaccga acgaagaagc aggtgcagca     720
   ctgtctccgc tgccggaagt cccgccgtgg accggtgaag ttagtccggc gcaacgtgaa     780
   gtggttccgc cgcaggttct gagcgaaccg aacgaagaag caggtgcagc actgtctccg     840
   ctgccggaag tcccgccgtg gaccggtgaa gttagtccgg cgcaacgtta agctt          895
<210> 18
   <211> 1117
   <212> DNA
   <213> Artificial sequence
<220>
   <223> B72(Q)R-(VSDL)8 (1117 bp)
<400> 18
   catatgaaag aagtgaagtc attgctgctg gatttacagt tgcttttgga gaaagttaaa    60
   aatcctgaga acctcaagct ctccaggatg catacatttg acttttacgt gcccaaggtt   120
   aacgctacag aattgaaaca tcttaaggcg ttactagaag aactcaaact tctagaggaa   180
   gtactaaatt tagctccaag caaaaacctg aacgtccagc gtgaagtggt tccgccgcag   240
   gttctgagcg aaccgaacga agaagcaggt gcagcactgt ctccgctgcc ggaagtcccg   300
   ccgtggaccg gtgaagttag tccggcgcaa cgtgaagtgg ttccgccgca ggttctgagc   360
   gaaccgaacg aagaagcagg tgcagcactg tctccgctgc cggaagtccc gccgtggacc   420
   ggtgaagtta gtccggcgca acgtgaagtg gttccgccgc aggttctgag cgaaccgaac   480
   gaagaagcag gtgcagcact gtctccgctg ccggaagtcc cgccgtggac cggtgaagtt   540
   agtccggcgc aacgtgaagt ggttccgccg caggttctga gcgaaccgaa cgaagaagca   600
   ggtgcagcac tgtctccgct gccggaagtc ccgccgtgga ccggtgaagt tagtccggcg   660
   caacgtgaag tggttccgcc gcaggttctg agcgaaccga acgaagaagc aggtgcagca   720
   ctgtctccgc tgccggaagt cccgccgtgg accggtgaag ttagtccggc gcaacgtgaa   780
   gtggttccgc cgcaggttct gagcgaaccg aacgaagaag caggtgcagc actgtctccg   840
   ctgccggaag tcccgccgtg gaccggtgaa gttagtccgg cgcaacgtga agtggttccg   900
   ccgcaggttc tgagcgaacc gaacgaagaa gcaggtgcag cactgtctcc gctgccggaa   960
   gtcccgccgt ggaccggtga agttagtccg gcgcaacgtg aagtggttcc gccgcaggtt  1020
   ctgagcgaac cgaacgaaga agcaggtgca gcactgtctc cgctgccgga agtcccgccg  1080
   tggaccggtg aagttagtcc ggcgcaacgt taagctt                           1117
<210> 19
   <211> 1339
   <212> DNA
   <213> Artificial sequence
<220>
   <223> B72(Q)R-(VSDL)10 (1339 bp)
<400> 19
   catatgaaag aagtgaagtc attgctgctg gatttacagt tgcttttgga gaaagttaaa      60
   aatcctgaga acctcaagct ctccaggatg catacatttg acttttacgt gcccaaggtt     120
   aacgctacag aattgaaaca tcttaaggcg ttactagaag aactcaaact tctagaggaa     180
   gtactaaatt tagctccaag caaaaacctg aacgtccagc gtgaagtggt tccgccgcag     240
   gttctgagcg aaccgaacga agaagcaggt gcagcactgt ctccgctgcc ggaagtcccg     300
   ccgtggaccg gtgaagttag tccggcgcaa cgtgaagtgg ttccgccgca ggttctgagc     360
   gaaccgaacg aagaagcagg tgcagcactg tctccgctgc cggaagtccc gccgtggacc     420
   ggtgaagtta gtccggcgca acgtgaagtg gttccgccgc aggttctgag cgaaccgaac     480
   gaagaagcag gtgcagcact gtctccgctg ccggaagtcc cgccgtggac cggtgaagtt     540
   agtccggcgc aacgtgaagt ggttccgccg caggttctga gcgaaccgaa cgaagaagca     600
   ggtgcagcac tgtctccgct gccggaagtc ccgccgtgga ccggtgaagt tagtccggcg     660
   caacgtgaag tggttccgcc gcaggttctg agcgaaccga acgaagaagc aggtgcagca     720
   ctgtctccgc tgccggaagt cccgccgtgg accggtgaag ttagtccggc gcaacgtgaa     780
   gtggttccgc cgcaggttct gagcgaaccg aacgaagaag caggtgcagc actgtctccg     840
   ctgccggaag tcccgccgtg gaccggtgaa gttagtccgg cgcaacgtga agtggttccg     900
   ccgcaggttc tgagcgaacc gaacgaagaa gcaggtgcag cactgtctcc gctgccggaa     960
   gtcccgccgt ggaccggtga agttagtccg gcgcaacgtg aagtggttcc gccgcaggtt    1020
   ctgagcgaac cgaacgaaga agcaggtgca gcactgtctc cgctgccgga agtcccgccg    1080
   tggaccggtg aagttagtcc ggcgcaacgt gaagtggttc cgccgcaggt tctgagcgaa    1140
   ccgaacgaag aagcaggtgc agcactgtct ccgctgccgg aagtcccgcc gtggaccggt    1200
   gaagttagtc cggcgcaacg tgaagtggtt ccgccgcagg ttctgagcga accgaacgaa    1260
   gaagcaggtg cagcactgtc tccgctgccg gaagtcccgc cgtggaccgg tgaagttagt    1320
   ccggcgcaac gttaagctt                                                 1339
<210> 20
   <211> 20
   <212> PRT
   <213> Artificial Sequence
<220>
   <223> wherein X is any amino acid except arginine or lysine
<220>
   <221> misc_feature
   <222> (4)..(4)
   <223> Xaa can be any naturally occurring amino acid
<220>
   <221> misc_feature
   <222> (9)..(9)
   <223> Xaa can be any naturally occurring amino acid
<220>
   <221> misc_feature
   <222> (11)..(11)
   <223> Xaa can be any naturally occurring amino acid
<220>
   <221> misc_feature
   <222> (13)..(13)
   <223> Xaa can be any naturally occurring amino acid
<400> 20
   Ser Ser Asp Xaa Ser Ala Leu Leu Xaa Ser Xaa Leu Xaa Ala Leu Leu
   1               5                   10                  15
   Thr Ala Pro Arg
               20

## Description

### TECHNICAL FIELD

The present invention relates to a method for producing a peptide, particularly a recombinant peptide. In a particular application, the method involves cleavage of a fusion polypeptide comprising concatemeric copies of a peptide.

### BACKGROUND

Recombinantly produced peptides are of increasing importance to industry and in clinical settings. Producing eukaryotic proteins recombinantly in various expression systems such as in bacterial, yeast, insect, plant or mammalian cells provides a low cost alternative to synthetic peptide production. However, recombinant expression is unpredictable and often problematic, as many recombinantly expressed peptides are not sufficiently soluble, are unstable, misfolded or inactive, and/or expressed at a low yield, and accordingly, are often not produced in a sufficiently high quality or yield to be of commercial value. It can be particularly difficult to express recombinantly expressed peptides with low molecular weight. Operably fusing the peptide to a suitable peptide fusion partner can enhance stability and solubility; however, it is generally not desirable to have a fusion partner present in peptides intended for therapeutic use.

Peptides can be cleaved by a large number of peptide cleaving agents including enzymatic cleaving agents (ie proteases) as well as chemical cleaving agents. Many peptide cleaving agents recognise and cleave peptides at a specific amino acid sequence or motif within the peptide. Peptides usually contain multiple copies of many different peptide cleavage sites. For example, the mature human interleukin (IL)-1β peptide (a 153 amino acid peptide, see Genbank Accession No AAA74137.1), is predicted to be cleaved by at least 20 different peptide cleaving agents. The mature IL-1β peptide sequence includes three predicted Arg-C proteinase cleavage sites, eight predicted Asp-N endopeptidase cleavage sites, six predicted cyanogen bromide cleavage sites, eight predicted formic acid cleavage sites, three predicted Clostripain cleavage sites, 17 predicted trypsin cleavage sites, etc. Accordingly, peptide cleaving agents are not generally useful during recombinant production of peptides as the peptide would be frequently cleaved into smaller fragments, which is undesirable.

The present inventors have realised that certain peptides are unusual in that they lack particular peptide cleavage sites, or contain a single copy of the particular peptide cleavage site located at a terminus of the peptide. For example, the present inventors have realised that the native vessel dilator (VSDL) peptide unusually contains a single trypsin cleavage site, which is located at the C terminus of the VSDL peptide.

Vessel dilator (VSDL) is a naturally occurring 37 amino acid (aa) peptide, which is produced *in vivo* following processing of the 126 amino acid atrial natriuretic peptide (ANP, also known as atrial natriuretic factor (ANF)) prohormone (proANP; Vesely, 2003). VSDL consists of amino acids 31-67 of the ANP prohormone. The main biological activity of VSDL is to regulate blood pressure and maintain plasma volume in healthy individuals by mediating natriuretic, diuretic and haemodynamic effects (Vesely, 2003). Investigations into the use of VSDL for the treatment of cardiac diseases such as congestive heart failure (CHF) have been conducted via both preclinical and human clinical studies. It has been shown that VSDL can significantly improve natriuretic, diuretic and haemodynamic parameters without any symptomatic side effects. VDSL is considered to be a safe and effective treatment for mediating beneficial haemodynamic effects with additional beneficial natriuretic, diuretic and renal effects, whilst regulating plasma volume and blood pressure (BP) within clinically acceptable ranges and without seriously adverse side effects. Accordingly, the administration of VSDL to patients with acute decompensated congestive heart failure (ADCHF) has been proposed. Moreover, VDSL has also been found to have anticancer effects (Skelton *et al.* 2011), and has been shown to have promise in the treatment of acute renal failure (Vesely, 2003). Accordingly, it will be appreciated that VSDL is a useful candidate for the treatment of various diseases. Synthetically produced VSDL based upon a native VSDL sequence has been utilised in clinical trials and has been advantageously shown to be useful in treating disease; however, synthetic production of peptides is an expensive process, particularly for peptides required on a commercial scale.

The present inventors have designed an elegant recombinant production system that utilises cleavage of peptides from a fusion polypeptide.

### SUMMARY

Thus, in a first aspect, the present invention provides a method of producing a VSDL recombinant peptide, the method comprising the following steps:
expressing a fusion polypeptide comprising concatemeric copies of the peptide wherein each concatemeric copy of the peptide is flanked by N-terminally located peptide cleavage site(s) and a C-terminally located peptide cleavage site and wherein said peptide cleavage sites are otherwise absent from said peptide; and
cleaving the fusion polypeptide at the peptide cleavage site(s) with a cleaving agent(s) that cleaves at the peptide cleavage site(s);
wherein the step of cleaving the fusion polypeptide releases said peptide from the fusion polypeptide, characterised in that:
   the VSDL peptide consists of a native peptide sequence comprising a C-terminal arginine residue;
   the C-terminally located peptide cleavage site of each concatemeric copy of the VSDL peptide consists of the C-terminal arginine residue; and
   the C-terminal arginine residue of each VSDL peptide except the last VSDL peptide in the fusion polypeptide additionally serves as the N-terminally located peptide cleavage site of a following VSDL peptide,
   wherein the VSDL peptide comprises or consists of the amino acid sequence according to SEQ ID NO: 2.

In an embodiment, the fusion polypeptide comprises from two to twenty (or more) concatemeric copies of the peptide. In an embodiment, the fusion polypeptide comprises three concatemeric copies of the peptide. In a further embodiment, the fusion polypeptide comprises ten concatemeric copies of the peptide.

The fusion polypeptide may comprise a leader fusion partner having a C-terminal peptide cleavage site. In a preferred embodiment, the leader fusion partner comprises a C-terminal arginine residue. In a particular embodiment, the leader fusion partner comprises the amino acid sequence according to SEQ ID NO: 3.

In an embodiment, the peptide cleavage site(s) is/are trypsin cleavage site(s). In an embodiment, the cleaving agent is trypsin or a trypsin-like enzyme.

In a second aspect, the present invention provides an expression construct comprising a nucleotide sequence encoding a leader fusion partner (having a C-terminal trypsin cleavage site) operably fused to at least one nucleotide sequence encoding two to twenty concatemeric copies a VSDL peptide (or a variant or modified peptide thereof), wherein the leader fusion partner has a C-terminal trypsin cleavage site, characterised in that:
the VSDL peptide consists of a native peptide sequence comprising a C-terminal arginine residue;
the C-terminally located peptide cleavage site of each concatemeric copy of the VSDL peptide consists of the C-terminal arginine residue; and
the C-terminal arginine residue of each VSDL peptide except the last VSDL peptide in the fusion polypeptide additionally serves as the N-terminally located peptide cleavage site of a following VSDL peptide,
wherein the VSDL peptide comprises or consists of the amino acid sequence according to SEQ ID NO: 2.

In another aspect, the present invention provides a host cell for recombinantly expressing a VSDL peptide (or a variant or modified peptide thereof), wherein the host cell comprises the expression construct according to the second aspect of the invention.

### BRIEF DESCRIPTION OF FIGURES

**Figure 1** provides the amino acid sequences of a VSDL peptide and amino acid sequences of VSDL 1-mer and 3-mer fusions with a leader fusion partner designated B72R; the italicised sequence represents the leader fusion partner (B72R) and the trypsin cleavage sites are indicated by arrows;
**Figure 2** provides the nucleotide sequence of B72R and B72(Q)R-VSDL gene fusions, including flanking restriction enzyme cleavage sites; protein coding regions are shown in bold, wherein the italicised sequences represent the nucleotide sequence of the leader fusion partner. The length of each gene fusion is shown in parentheses;
**Figure 3** provides schematic maps of plasmids for the expression of B72R-VSDL and B72R-based fusion polypeptides comprising 3, 6, 8 and 10 tandem repeats of VSDL. Plasmid functions are origin of replication (ColE1 ori), lac repressor gene (lacI), trc and tac promoters, VSDL gene fusion, transcription terminator (rrnBT1T2) and a kanamycin resistance marker (Km);
**Figure 4** provides an image of SDS-PAGE analysis of B72R-VSDL and B72R-(VSDL)₃ expression; fractionated culture samples (P: Pellet/Insoluble fraction, WCL: Whole Cell Lysate and S: Supernatant/Soluble fraction) were run on reducing 4-12% NuPAGE MES gels (Invitrogen Corporation, Carlsbad CA, United States of America) and stained with SimplyBlue™ Safe (Invitrogen). SeeBlue® pre-stained protein standard (Invitrogen) was used as molecular weight marker;
**Figure 5** provides an image of SDS-PAGE analysis of B72(Q)R-VSDL fusion polypeptide expression; designated culture samples were normalised and lysates run on reducing 4-12% NuPAGE MES gels stained with SimplyBlue™ Safe, against the SeeBlue® pre-stained protein standard as the molecular weight marker;
**Figure 6** provides an image of SDS-PAGE analysis of B72R-VSDL and B72R-(VSDL)₃ expression following fed-batch cultivation; designated culture samples were normalised and lysates run on reducing 4-12% NuPAGE MES gels stained with SimplyBlue™ Safe, against the SeeBlue® prestained protein standard as the molecular weight marker;
**Figure 7** provides graphical results showing the effect of TrypZean® (Sigma-Aldrich Co, St Louis Mo, United States of America) addition on the amount of VSDL generated from B72R-(VSDL)₃ lysate (3 g/L fusion polypeptide) after overnight digestion at room temperature;
**Figure 8** provides a trace showing chromatographic separation (Amberchrom CG300C; Rohm and Haas Company, Philadelphia PA, United States of America) of recombinant VSDL from digested lysate; solid line = UV 280 absorption and the broken line = conductivity;
**Figure 9** provides a trace showing chromatographic separation (Fractogel TMAE; Merck Inc, Whitehouse Station NJ, United States of America) of recombinant VSDL from digested lysate; solid line = UV 280 absorption and broken line = conductivity;
**Figure 10** shows the UV 280 absorption profiles for the synthetic VSDL peptide and recombinant VSDL (right) as measured by a Cary 50 spectrophotometer (Varian Inc, Palo Alto CA, United States of America);
**Figure 11** provides a trace showing chromatographic separation (modified Amberchrom CG300C protocol) of recombinant VSDL from a digested lysate, the box represents pooled eluate fractions; and

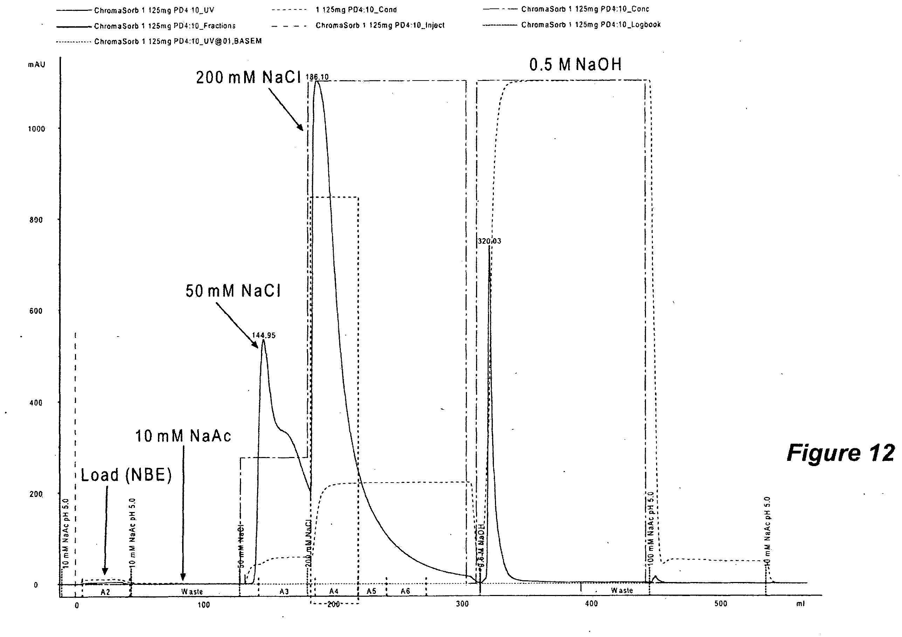

**Figure 12** provides a trace showing separation of recombinant VSDL from a digested lysate following Chromasorb membrane filtration, wherein the boxes represent various pooled eluate fractions.

### DETAILED DESCRIPTION

The present inventors have realised that, advantageously, the amino acid sequences of certain peptides unusually lack particular peptide cleavage sites, with the exception of a single peptide cleavage site located at one terminus of the peptide, or can be engineered to have such a sequence. The present inventors have also realised that by expressing the peptide in the context of a fusion polypeptide, a further peptide cleavage site may be provided at the opposite terminus of the peptide, such that cleavage of the expressed fusion polypeptide releases the peptide from the fusion polypeptide. Such a production system could advantageously provide a means of recombinantly producing a peptide of interest in sufficient quality and quantity for therapeutic use.

The term "recombinant" will be understood by the person skilled in the art as referring to biological material containing or derived from genetic material of more than one origin, for example, a recombinant peptide would generally be understood to be derived from a polynucleotide molecule encoding the peptide of a first origin that is expressed or produced in a suitable host cell of a second origin. A wide range of expression systems that can produce recombinant peptides are known, for example, in cell cultures under conditions suitable for the expression of the particular peptide. The cellular hosts can include cells of bacteria (eg *Escherichia coli, Streptomyces sp., Bacillus sp., Pseudomonas sp., Staphylococcus sp.* (eg *S. typhimurium* etc), etc), fungal cells such as yeast cells (eg *Saccharomyces cerevisiae, Pichia pastoris,* etc), molds or filamentous fungi (eg *Aspergillus sp.*), insect cells (such as Drosophila S2 and *Spodoptera frugiperda* (such as Spodoptera Sf9 cells), and including baculovirus-assisted systems), mammalian cells (numerous cell lines are suitable including Chinese Hampster Ovary (CHO) cells, monkey kidney (COS) cells, human embryonic kidney (HEK) cells, baby hampster kidney (BHK) cells, murine melanoma cells, etc), and plant cells. Recombinant peptides can alternatively be expressed in transgenic plants and animals. In the present invention, it is preferred that the step of expressing the fusion polypeptide is conducted using *E. coli* as an expression host.

The term "peptide" will be well understood by the person skilled in the art as referring to a single linear polymer chain of amino acids bonded together by peptide bonds, with the first amino acid being at the amino (N) terminus and the last amino acid being at the carboxyl (C) terminus. In the context of the present invention, it is to be understood that the term "peptide" as used herein is intended to refer to a peptide of interest which is to be expressed and released intact from the fusion polypeptide following cleavage of the fusion polypeptide. The.peptide may be a full length peptide (ie the full length of the peptide as would be transcribed from the encoding nucleotide sequence as would be understood by a person skilled in the art). However, the peptide may also be derived from a larger peptide or polypeptide, for example, the peptide may be the mature peptide portion of a pre-pro-protein, or be a portion of a polypeptide that can be processed into a number of smaller peptides, or be a fragment of a larger peptide, etc. Preferably, the peptide may be translated from the encoding nucleotide sequence into its final form, that is, the peptide does not undergo any additional post-translational processing other than the cleavage by the cleaving agent(s) in accordance with the present invention. However, the present invention does not intend to exclude embodiments wherein the peptide is a portion of a larger peptide, such as a pre-pro-protein or a polypeptide that comprises an amino acid sequence that can be processed into a number of smaller peptides (eg the portion(s) of the larger peptide are removed by processing (eg by cleavage) following expression, wherein the processing is in addition to the cleavage according to the present invention). The peptide may be a native peptide or a peptide that has been modified as described below.

The person skilled in the art will appreciate that the term "amino side" or "amino direction" when used in connection with a peptide is referring to the direction toward the N terminus of the peptide, and likewise, the terms "carboxyl side" or "carboxyl direction" when used in connection with a peptide is referring to the direction toward the C terminus of the peptide.

The term "fusion polypeptide" as used herein is intended to refer to at least two peptides fused together in a single linear polymer chain of amino acids bonded together by peptide bonds, such that the C-terminal residue of a first peptide is adjacent the N-terminal residue of a second peptide (optionally separated by a short linker sequence). The person skilled in the art will appreciate that a number of peptides can be fused together in this manner, and in this event, the C-terminal residue of the second peptide may be adjacent the N-terminal residue of a third peptide, and so on.

The term "peptide cleavage site" will be well understood by the person skilled in the art as referring to the site where the peptide backbone is cleaved by a cleaving agent, wherein that site is associated with a particular amino acid or a particular sequence of amino acids that serves as a recognition site for the cleaving agent. In the case of a particular recognised sequence of amino acids, a cleaving agent may cleave the peptide backbone at a site within that sequence, at the C-terminal end or the N-terminal end of the sequence, or externally to the sequence, that is, a number of residues away in the carboxyl or, alternatively, amino direction from the sequence.

In accordance with the present invention, said peptide is "flanked by one or more peptide cleavage sites". This phrase is intended to mean that peptide cleavage sites are located within the fusion polypeptide at at least one end of the peptide and, more preferably, at both ends of the peptide (eg one N-terminally located and one C-terminally located with respect to each copy of the peptide), such that said peptide can be released from the fusion polypeptide upon cleavage of the fusion polypeptide with a cleaving agent that cleaves at the peptide cleavage sites.

The cleaving agent may be any suitable cleaving agent known to the person skilled in the art that cleaves peptides in a specific manner, that is, at a specific peptide cleavage site associated with a particular amino acid or a particular sequence of amino acids. The cleaving agent may be a chemical cleaving agent or an enzymatic cleaving agent. A number of cleaving agents that may be used in the present invention are listed in Table 1.

**Table 1 Peptide cleaving agents and their respective peptide cleavage sites**

| **Cleaving agents** | **Cleavage sites** |
|---|---|
| Arg-C proteinase | R↓ |
| Asp-N endopeptidase | ↓N |
| Asp-N endopeptidase + N-terminal Glu | ↓N, ↓E |
| Clostripain (Clostridiopeptidase B) | R↓ |
| Enteropeptidase (enterokinase) | DDDDK↓ (SEQ ID NO: 4) |
| Thrombin | R↓, but preferentially LVPR↓GS |
| Tobacco etch virus protease | EXXYXQ↓(G/S) (SEQ ID NO: 5) or XYXQ↓(G/S) (SEQ ID NO: 6), where X is any amino acid |
| Trypsin | K↓ or R↓ |
| BNPS-Skatole [2-(2-nitrophenylsulfenyl)-3-methylindole] | Y↓ |
| *N*-chlorosuccinimide (NCS) | W↓ |
| *N*-bromosuccinimide (NBS) | W↓ |
| Cyanogen bromide | M↓ |
| Formic Acid | D↓ |

Herein, peptide cleavage sites are shown by an arrow located at the amino side or carboxyl side of a single, particular amino acid serving as a recognition site for a cleaving agent, or by an arrow located at the amino side, carboxyl side or within a particular sequence of amino acids serving as a recognition site for a cleaving agent. The three-letter or one-letter amino acid codes used in relation to the peptide cleavage sites (and all amino acid sequences herein) are shown in Table 2.

**Table 2 Amino Acid Codes**

| **Amino acid** | **Three letter code** | **One letter code** |
|---|---|---|
| Alanine | Ala | A |
| Arginine | Arg | R |
| Asparagine | Asn | N |
| Aspartic acid (Aspartate) | Asp | D |
| Cysteine | Cys | C |
| Glutamine | Gln | Q |
| Glutamic acid (Glutamate) | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

In a preferred embodiment, the cleaving agent is typsin or a trypsin-like enzyme (ie an enzyme that cleaves at a typsin cleavage site).

Other cleaving agents may, however, also be suitable for use in the present invention including, for example, caspase1, caspase2, caspase3, caspase4, caspase5, caspase6, caspase7, caspase8, caspase9, caspase10, chymotrypsin-high specificity, chymotrypsin-low specificity, Factor Xa, formic acid, glutamyl endopeptidase, granzyme B, hydroxylamine, iodosobenzoic acid, LysC, LysN, NTCB (2-nitro-5-thiocyanobenzoic acid), pepsin (pH 1.3), pepsin (pH>2), proline-endopeptidase, proteinase K, Staphylococcal peptidase I, thermolysin, ubiquitin protease, etc. The person skilled in the art will appreciate that other cleaving agents may be used in accordance with the present invention, and would be well aware of ways of determining the peptide cleavage site for a suitable cleaving agent, in addition to determining what conditions are required for cleavage with a particular cleaving agent.

In an embodiment, a peptide cleavage site is provided at both ends of the peptide and may require two cleaving agents to release the peptide from the fusion polypeptide.

The present inventors have realised that the fusion polypeptide advantageously comprises concatemeric copies of the peptide, wherein each copy of the peptide is flanked by one or more peptide cleavage sites.

The phrase "concatemeric copies of the peptide" is to be understood as referring to more than one peptide operably linked in a tandem head-to-tail arrangement such that the C-terminal residue of a first peptide is adjacent the N-terminal residue of the second peptide. In some embodiments, wherein more than two concatemeric peptides are contemplated, the C-terminal residue of the second peptide is adjacent the N-terminal of a third peptide, and so on. Preferably, the C-terminal residue of one peptide is immediately adjacent the N-terminal residue of the following peptide. However, it will be apparent to the person skilled in the art that peptides could be joined by a short linker sequence that could potentially be used to link the copies of the peptide together. It is to be understood that such an embodiment falls within the scope of the present invention.

Any number of copies of the peptide may be concatemerically arranged within the fusion polypeptide, providing that the fusion polypeptide is expressed to a satisfactory level. In an embodiment, the fusion polypeptide comprises from two to twenty (or more) concatemeric copies of the peptide. For example, the fusion polypeptide may comprise two, three, four, five, six, seven, eight, nine, ten, eleven, twelve, thirteen, fourteen, fifteen, sixteen, seventeen, eighteen, nineteen or twenty concatametric copies of the peptide. In an embodiment, the fusion polypeptide comprises from two to ten concatemeric peptides.

In one embodiment, the fusion polypeptide comprises three concatemeric copies of the peptide. In another embodiment, the fusion polypeptide comprises ten concatemeric copies of the peptide. However, where there are a particular number of copies in the fusion polypeptide, for example, three concatemeric copies of the peptide, it is to be understood that there is a total of three of the peptide in the polypeptide. Accordingly, "three concatemeric copies of the peptide" is not intended to mean that there is an original peptide in addition to three "copies" of the peptide. Fusion polypeptides comprising concatemeric peptides (and the polynucleotide constructs encoding said fusion polypeptides) are additionally referred to herein as, for example, a "3-mer" when three concatemeric peptides are present, a "6-mer" when six concatemeric peptides are present, etc. Likewise, a fusion polypeptide comprising a single peptide (and the polynucleotide constructs encoding said fusion polypeptide) is additionally referred to herein as a "1-mer".

In accordance with the present invention, said peptide is "flanked by one or more peptide cleavage sites". In the context of concatermeric copies of the peptide, this phrase is intended to mean that peptide cleavage sites are located within the fusion polypeptide at least one end of every copy of the peptide and, more preferably, at both ends of the peptide (eg one N-terminally located and one C-terminally located with respect to each copy of the peptide), such that said peptide can be released from the fusion polypeptide upon cleavage of the fusion polypeptide with a cleaving agent that cleaves at the peptide cleavage sites.

In a preferred embodiment, a peptide cleavage site is provided at both ends of each copy of the peptide by a single peptide cleavage site between each peptide copy, the arrangement being such that peptide cleavage by a single cleaving agent at said site simultaneously produces the C-terminus of one copy of the peptide and the N-terminus of the adjacent copy of the peptide. In this embodiment, it is preferred that the single peptide cleavage site be of the same type (eg R↓) between all copies of the peptide.

The method of the the present invention may be particularly suitable for producing any peptide that is characterised in that it has no internal peptide cleavage sites.

The peptide of the present invention has a native sequence. The term "native" in this context is intended to mean that the sequence of the peptide is as found in nature, that is, not modified, for example, to remove peptide cleavage sites or otherwise to introduce amino acid substitutions. The peptide may, however, have a truncated native sequence or a native variant sequence.

As disclosed herein, the peptide may be modified. A modified peptide suitable for use in the present invention may comprise a derivative, variant or mimetic of the native peptide which includes minor variation(s) in the amino acid sequence providing that such variation(s) do not result in any substantial decrease or variation in biological activity (ie as compared to the native peptide), and do not introduce a further peptide cleavage site(s) (preferably, no further trypsin cleavage site(s)). These variation(s) may include conservative amino acid substitutions such as: Gly, Ala, Val, Ile, Leu, Met; Asp, Glu, Asn, Gln; Ser, Thr; Lys, Arg, His; Phe, Tyr, Trp, His; and Pro, Nα-alkylamino acids; and non-conservative amino acid substitutions. Table 3 details common conservative amino acid substitutions.

**Table 3 Exemplary conservative amino acid substitutions**

| | **Conservative Substitutions** |
|---|---|
| Ala | Val*, Leu, Ile |
| Arg | Lys* |
| Asn | Gln*, His, Asp |
| Asp | Glu*, Asn |
| Cys | Ser |
| Gln | Asn*, His, |
| Glu | Asp*, γ-carboxyglutamic acid (Gla) |
| Gly | Pro |
| His | Asn, Gln, |
| Ile | Leu*, Val, Met, Ala, Phe, norleucine (Nle) |
| Leu | Nle, Ile*, Val, Met, Ala, Phe |
| Lys | Arg*, Gln, Asn, ornithine (Orn) |
| Met | Leu*, Ile, Phe, Nle |
| Phe | Leu*, Val, Ile, Ala |
| Pro | Gly*, hydroxyproline (Hyp), Ser, Thr |
| Ser | Thr |
| Thr | Ser |
| Trp | Tyr |
| Tyr | Trp, Phe*, Thr, Ser |
| Val | He, Leu*, Met, Phe, Ala, Nle |

| | |
|---|---|
| *indicates preferred conservative substitutions | |

Suitable modified peptides may be obtained using any of the methods well known to the person skilled in the art. For example, mimetics of peptides can be designed using any of the methods well known to the person skilled in the art based upon amino acid sequences in the absence of secondary and tertiary structural information (Kirshenbaum *et al*., 1999).

Moreover, the person skilled in the art will appreciate that it may be possible to engineer peptides to remove existing internal peptide cleavage sites (eg by substitution or deletion of appropriate amino acids). For example, the amino acid sequence of native human glucagon-like peptide (GLP)-1 (derived from amino acids 98 to 127 of human glucagon preproprotein, which has NCBI Reference NP_002045) contains a C-terminal arginine (R) residue and two internal lysine (K) residues. The two internal lysine residues can be modified, for example, to glutamine (Q) or asparagine (N), which removes the internal trypsin cleavage sites. In another example, some membrane proteins have low numbers of arginine and lysine residues compared to cytosolic proteins, and such residues could be removed by modification to create peptides with no internal peptide cleavage sites.

In an embodiment, each concatemeric copy of the peptide is identical to every other concatemeric copy of the peptide. However, in an alternative embodiment, different variants of the peptide may be expressed within the concatemer. That is, the term "concatemeric copies of the peptide" is intended to include situations where one (or more) "concatemeric copies" of the peptide are natural variants or modified peptides compared to another concatemeric copy of the peptide. For example, one concatemeric copy of the peptide may comprise a native sequence, while a further concatemeric copy of the peptide comprises a natural variant of the peptide, etc. In another example, different concatemeric copies of the peptide may comprise different native or modified amino acid sequences so as to generate a different amino acid or sequence of amino acids to serve as a recognition site for the cleaving agent.

In an embodiment, the peptide cleavage site is a trypsin cleavage site.

The term "trypsin cleavage site" will be understood by the person skilled in the art as referring to the site where the peptide backbone is cleaved by trypsin, wherein that site is associated with a particular amino acid or a particular sequence of amino acids that serves as a recognition site for trypsin. Trypsin tends to cleave peptides at the carboxyl side of lysine (K) or arginine (R) amino acid residues, except when they are immediately followed by proline (P). However, the exact amino acid residues surrounding the lysine or arginine residue may affect the efficiency of trypsin cleavage. Examples of recognition sites for trypsin include, for example, DR↓E, QR↓E, SK↓N, etc, where the arrow designates the peptide cleavage site (ie the site where the peptide backbone is cleaved). However, the person skilled in the art will appreciate that most arginine or lysine residues may serve as the site for carboxyl side cleavage by trypsin.

The step of cleaving can be achieved by, for example, incubating the fusion polypeptide with a cleaving agent that cleaves at trypsin cleavage sites (ie at the carboxyl side of an arginine or lysine residue) under appropriate conditions for cleavage to occur, as would be understood by the person skilled in the art.

In an embodiment, the cleaving agent is trypsin. Trypsin is a serine protease and is frequently used in research laboratories to cleave peptides. Trypsin for use in accordance with the present invention may be isolated from a number of different species (including bovine, porcine, human etc), all of which is commercially available, and/or recombinantly produced trypsin (including bovine, porcine, etc), which is also readily available, may be used. Further, trypsin can be recombinantly produced in plant cells, such as corn cells. TrypZean® (eg bovine TrypZean, Sigma-Aldrich) is an example of plant-produced recombinant trypsin. Accordingly, in an embodiment, the cleaving agent is trypsin that has been recombinantly produced by plant cells. Plant-produced recombinant trypsin is particularly useful for cleaving peptides intended for therapeutic use to reduce the possibility of undesirable animal-derived contaminants being present in the final peptide product. However, other trypsin products that are produced in a manner such that the trypsin is essentially free of (or has acceptably low levels of) animal contaminants may be useful for peptides produced for therapeutic use. For example, the cleaving agent may be trypsin that has been produced by yeast cells. Preferably, the cleaving agent is recombinant trypsin.

However, the person skilled in the art will appreciate that there are a number of other enzymes that have trypsin or trypsin-like activity (ie a trypsin-like enzyme that cleaves at a trypsin cleavage site) that may also be suitable for cleaving a peptide at a trypsin cleavage site under certain circumstances in accordance with the present invention. For example, thrombin is a trypsin-like serine protease that preferentially recognises the amino acid sequence LVPR↓GS (SEQ ID NO: 7) and cleaves at the peptide cleavage site between the arginine and glycine residues (ie at the carboxyl side of an arginine (R) residue). Accordingly, thrombin will cleave peptides at some trypsin cleavage sites. In another example, clostripain (also known as Endoproteinase Arg-C and Clostridiopeptidase B) primarily cleaves at arginine residues, but may also cleave at lysine residues to a lesser degree. Accordingly, clostripain will cleave at trypsin cleavage sites. The person skilled in the art will appreciate that there may be other cleaving agents that are also suitable for cleaving peptides at trypsin cleavage sites in accordance with the present invention. Thus, in an embodiment, the cleaving agent is trypsin or a trypsin-like enzyme. In an embodiment, the cleaving agent is recombinant trypsin or a recombinant trypsin-like enzyme.

Trypsin digestion time can be varied as required. For example, the fusion polypeptide may be digested by the trypsin or trypsin-like enzyme for 5 minutes, 1 hour, 2 hours, 6 hours, or overnight, etc.

As disclosed herein, the peptide may comprise a C-terminal trypsin cleavage site. The peptide may comprise an amino acid sequence according to the formula

X₁-X₂- ... Xₙ₋₁-Xₙ-(R/K) (SEQ ID NO: 1);

wherein X is any amino acid except arginine or lysine, R/K is arginine or lysine, and n is any suitable number of amino acids.

The peptide produced may be for therapeutic use. As such, the peptide may comprise a cytokine or hormone (including a hormone rereleased from a pro-hormone molecule) or an epitope or antigen (eg for use as a vaccine).

The peptide may be of, for example, 3 to 100 amino acids in length, 5 to 50 amino acids in length, or of 10 to 45 amino acids in length. The peptide may be of 30 to 40 amino acids in length, including for example, peptides of 33, 34, 35, 36 and 37 amino acids in length. The peptide may have no internal trypsin cleavage site(s).

As disclosed herein, the peptide is selected from peptides derived from atrial natriuretic peptide (ANP), as modified, if necessary, to remove internal peptide cleavage site(s). The peptide is vessel dilator peptide (VSDL). The peptide may be modified kaliuretic peptide (KP) that has no internal peptide cleavage sites, and no internal trypsin cleavage sites (ie with the exception of the C-terminal arginine, all arginine and lysine residues have been targeted for amino acid substitution, preferably by conservative amino acid substitution with histidine (H)).

Thus, the peptide may be a modified KP peptide (with no internal trypsin cleavage sites), and preferably a modified human KP comprising or consisting of the following amino acid sequence (derived from residues 79-98 of human atrial natriuretic prohormone (proANP)):
Ser-Ser-Asp-Xₐ-Ser-Ala-Leu-Leu-X_{b}-Ser-X_{c}-Leu-X_{d}-Ala-Leu-Leu-Thr-Ala-Pro-Arg (SEQ ID NO: 20);
wherein X is any amino acid except arginine or lysine.

The peptide is a VSDL peptide or a variant or modified peptide thereof. As used herein, a variant of VSDL may be a natural variant. Otherwise, a variant VSDL may be a modified VSDL peptide. Modified VSDL peptides include not only variant peptides (ie non-natural variants), but also derivatives and mimetics of a native VSDL peptide which include minor variations in the amino acid sequence that do not result in any substantial decrease or variation in biological activity (eg shows no more than a 10% decrease or variation in biological activity of the VSDL peptide from which it is derived, as measured by the *in vitro* vasodilation assay (using aortic strips) described by DL Vesely in United States Patent No 5,691,310 or assay for increased cyclic GMP levels described by DL Vesely (Vesely *et al.* 1987), and do not comprise or introduce a further peptide cleavage site (preferably, no further trypsin cleavage site(s)) recognised by the said cleaving agent. These variations may include conservative amino acid substitutions such as detailed above in Table 3. Some specific examples of suitable amino acid substitutions within the VSDL peptide may include Pro→Gln (especially at position 41 of proANP; ie position 10 of the VSDL peptide), Thr→Ala (especially at position 59 of proANP; ie position 28 of the VSDL peptide), Glu→Asp (especially at position 61 of proANP, ie position 30 of the VSDL peptide), and Ser→Asn (especially at position 63 of proANP, ie position 32 of the VSDL peptide). The peptide is a VSDL peptide with a native sequence, such as the native amino acid sequence of human VSDL (derived from residues 31-67 of human proANP) as follows:

The peptide is a VSDL peptide comprising or consisting of the amino acid sequence according to SEQ ID NO: 2.

The VSDL peptide (or a variant or modified peptide thereof) comprises a C-terminal arginine residue. For example, the native VSDL peptides described herein comprise a C-terminal arginine residue, but have no internal arginine or lysine residues. Accordingly, the C-terminal arginine residue may serve as a C-terminally located peptide cleavage site. That is, peptide cleaving agents that cleave at the carboxyl side of arginine residues, such as trypsin and trypsin-like enzymes, will cleave the VSDL peptide (or a variant or modified peptide thereof) from the fusion polypeptide (eg from an adjacent copy of the VSDL peptide or a variant or modified peptide thereof) precisely at the C terminus.

However, the human VSDL peptide (or a variant or modified peptide thereof), in addition to lacking internal arginine residues, additionally lacks aspartic acid, cysteine, histidine, isoleucine, lysine, methionine, phenylalanine or tyrosine residues. Accordingly, it may be possible to utilise peptide cleavages sites that cleave at one of these sites in accordance with the present invention, that is, for example, at a peptide cleavage site located N-terminally with respect to the VSDL peptide (or a variant or modified peptide thereof), as described below. Alternatively or additionally, the C-terminal arginine residue of the VSDL peptide (or a variant or modified peptide thereof) could be substituted with another of the aspartic acid, cysteine, histidine, isoleucine, lysine, methionine, phenylalanine or tyrosine residues, such that the peptide could be cleaved from the fusion polypeptide by a cleaving agent that cleaves at one of these residues. The person skilled in the art will appreciate that this embodiment may alternatively be applied to other suitable peptides as described herein.

In an embodiment, the fusion polypeptide comprises one or more amino acid(s) located N-terminally with respect of the VSDL peptide (or a variant or modified peptide thereof), including a peptide cleavage site. Preferably, the N-terminally located peptide cleavage site is located immediately adjacent the N terminus of the VSDL peptide (or a variant or modified peptide thereof). In a preferred embodiment of the invention, the fusion polypeptide comprises a leader fusion partner having a C-terminal peptide cleavage site that provides an N-terminally located peptide cleavage site to the VSDL peptide (or a variant or modified peptide thereof).

In an embodiment, the VSDL peptide (or a variant or modified peptide thereof) is "flanked by trypsin cleavage sites". This is to be understood as meaning that a trypsin cleavage site is located within the fusion polypeptide at both ends of the VSDL peptide (ie one at the N-terminal end and one at the C-terminal end of the VSDL peptide) or a variant or modified peptide thereof, such that the VSDL peptide (or a variant or modified peptide thereof) can be released from the fusion polypeptide upon digestion with an enzyme that cleaves at trypsin cleavage sites. The person skilled in the art will appreciate that as cleavage occurs on the carboxyl side of the arginine or lysine residue of the trypsin cleavage site, the arginine or lysine residue will be excluded from the VSDL peptide (or a variant or modified peptide thereof) as released, whereas the C-terminally located trypsin cleavage site will mean that the arginine or lysine will be included in the VSDL peptide (or a variant or modified peptide thereof) as released (ie the arginine or lysine residue will form the C-terminal residue of the VSDL peptide as released).

Preferably, the VSDL peptide (or a variant or modified peptide thereof) released from the fusion polypeptide is intact (that is, the full length VSDL peptide); however, it is to be understood that the fusion polypeptide may be designed to release truncated or extended variants of the VSDL peptide (or a variant or modified peptide thereof) by varying the position of the trypsin cleavage sites. For example, the invention encompasses the expression of a fusion polypeptide wherein the N-terminally located trypsin cleavage site is located further upstream (ie in the amino direction) of the VSDL peptide (or a variant or modified peptide thereof) under certain circumstances, generating an N-terminally extended VSDL peptide upon enzymatic cleavage. Similarly, a C-terminally extended VSDL peptide (or a variant or modified peptide thereof) can be designed to include additional amino acids prior to the C-terminal trypsin cleavage site. Alternatively, a truncated VSDL peptide (or a variant or modified peptide thereof) can be designed by the deletion of amino acids as would be understood by the person skilled in the art.

The fusion polypeptide comprises concatemeric copies of the VSDL peptide (ie concatemeric repeats of the VSDL peptide) or a variant or modified peptide thereof. Preferably, each copy of the VSDL peptide (or a variant or modified peptide thereof) has a peptide cleavage site (eg a trypsin cleavage site) at the C terminus of each VSDL peptide (or a variant or modified peptide thereof), such that the peptide cleavage site additionally serves as the N-terminally located peptide cleavage site for the following VSDL peptide (or a variant or modified peptide thereof) in the concatemeric arrangement (except for the last VSDL peptide in the fusion polypeptide), such that each VSDL peptide (or a variant or modified peptide thereof) is flanked by peptide cleavage sites in accordance with the present invention. This arrangement advantageously results in each VSDL peptide (or a variant or modified peptide thereof) being individually released from the fusion polypeptide upon cleavage with a cleaving agent.

Any number of copies of the VSDL peptide (or a variant or modified peptide thereof) may be concatemerically arranged within the fusion polypeptide, providing that the fusion polypeptide is expressed to a satisfactory level. In an embodiment, the fusion polypeptide is expressed at at least 0.01 g/L, but preferably, the fusion polypeptide is expressed at at least 0.5 g/L, more preferably, at least 1 g/L, more preferably at least 2 g/L, more preferably 5 g/L, or more preferably 10 g/L. Preferably, the fusion polypeptide comprises from two to twenty (or more) concatemeric copies of the VSDL peptide (or a variant or modified peptide thereof). For example, the fusion polypeptide may comprise two, three, four, five, six, seven, eight, nine, ten eleven, twelve, thirteen, fourteen, fifteen, sixteen, seventeen, eighteen, nineteen or twenty concatemeric copies of the VSDL peptide (or a variant or modified peptide thereof). In an embodiment, the fusion polypeptide comprises from two to ten concatemeric copies of the VSDL peptide (or a variant or modified peptide thereof). In one preferred embodiment, the fusion polypeptide comprises three concatemeric copies of the VSDL peptide (or a variant or modified peptide thereof). In another embodiment, the fusion polypeptide comprises ten concatemeric copies of the VSDL peptide (or a variant or modified peptide thereof). In some embodiments, different variants of the VSDL peptide (or a variant or modified peptide thereof) may be expressed concatemerically.

Fusion polypeptides comprising concatemeric VSDL peptides (and the polynucleotide constructs encoding said fusion polypeptides), or a variant or modified peptide thereof, are additionally referred to herein as, for example, a "3-mer" when three concatemeric VSDL peptides (or a variant or modified peptide thereof) are present, a "6-mer" when six concatemeric VSDL peptides (or a variant or modified peptide thereof) are present, etc. Likewise, a fusion polypeptide comprising a single VSDL peptide, or a variant or modified peptide thereof (and the polynucleotide constructs encoding said fusion polypeptide) is additionally referred to herein as a "1-mer".

In embodiments of the present invention wherein the peptide comprises a C-terminal peptide cleavage site, the present invention is intended to include within its scope the situation wherein the first peptide in a series of concatemeric peptides is not released from the fusion polypeptide as it does not have a N-terminal peptide cleavage site. Likewise, in embodiments of the present invention wherein the peptide comprises an N terminal peptide cleavage site, the present invention is intended to include within its scope the situation wherein the last peptide in a series of concatemeric peptides is not released from the fusion polypeptide as it does not have a C-terminal peptide cleavage site. However, preferably, the fusion polypeptide further comprises a peptide cleavage site (ie located N-terminally with respect to concatemeric copies of the peptide comprising a C-terminal peptide cleavage site, or alternatively, located C-terminally with respect to concatemeric copies of the peptide comprising a N-terminal peptide cleavage site) such that each concatemeric copy of the peptide can be released from the fusion polypeptide.

In an embodiment, the fusion polypeptide may comprise a fusion partner. Advantageously, a fusion partner may also provide the fusion polypeptide with increased solubility, stability, activity (eg by reducing misfolding) and/or enhance expression yield. Preferably, the fusion partner provides a peptide cleavage site adjacent to one end of an adjacent peptide, such that, when the peptide comprises or is provided with a peptide cleavage site at its other end, the peptide is flanked by peptide cleavage sites in accordance with the present invention. In an embodiment, the fusion polypeptide may comprise a different peptide cleavage site compared with the peptide. In this case, two cleaving agents may be required to release the peptide from the fusion polypeptide.

The fusion polypeptide may comprise a trailing fusion partner, that is, the trailing fusion partner is located C-terminally with respect to the peptide or concatemeric copies of the peptide. The trailing fusion partner may comprise an N-terminal peptide cleavages site that provides a C-terminally located peptide cleavage site to a peptide (for example, to a peptide that comprises an N-terminally located peptide cleavage site, such that the peptide is flanked by peptide cleavage sites in accordance with the present invention). Accordingly, the trailing fusion peptide may comprise an N-terminal peptide cleavage site. The person skilled in the art would appreciate that such an N-terminal peptide cleavage site of a trailing fusion partner could serve as a C-terminally located peptide cleavage site for a leading peptide (that is, located N-terminally with respect of the trailing fusion partner).

The fusion polypeptide may comprise a leader fusion partner, the leader fusion partner is located N-terminally with respect to the peptide or concatemeric copies of the peptide. The leader fusion partner may comprise a C-terminal peptide cleavage site that provides a N-terminally located peptide cleavage site to a peptide (for example, to a peptide that comprises an C-terminally located peptide cleavage site, such that the peptide is flanked by peptide cleavage sites in accordance with the present invention). Accordingly, in an embodiment, the leader fusion peptide comprises a C-terminal peptide cleavage site. The person skilled in the art would appreciate that such a C-terminal peptide cleavage site of a leader fusion partner could serve as an N-terminally located peptide cleavage site for a following peptide (that is, located C-terminally with respect of the leader fusion partner).

Any suitable peptide known to the person skilled in the art may be the fusion partner, providing the resulting peptide is expressed in sufficient quality and/or yield. Examples of known fusion partners include ubiquitin, staphylococcal protein A, thioredoxin, maltose binding protein, glutathione-s-transferase, prochymosin, beta-galactosidase, gp 55 from T4, etc. Known recombinant tags may also provide a fusion partner, including His tag, Btag, FLAG, c-myc tag, protein C tag, S-tag, methionine, etc. The fusion partner can optionally contain additional peptide cleavage sites, resulting in the fusion partner being cleaved into a number of small peptides upon cleavage of the fusion polypeptide, which may aid purification of the desired peptide.

In an embodiment, the fusion polypeptide comprises a leader fusion polypeptide that is derived from interleukin (IL)-2, for example, bovine IL-2. More preferably, the leader fusion partner is a 72 amino acid fragment derived from bovine IL-2, referred to as B72R herein, which has the following amino acid sequence:

Accordingly, in an embodiment, the leader fusion partner is B72R.

In an embodiment, the penultimate aspartic acid residue of the B72R fusion partner is substituted for a glutamine residue (referred to as B72(Q)R herein), having the following amino acid sequence:

Accordingly, in an embodiment, the leader fusion partner is B72(Q)R.

In preferred embodiments, the leader fusion partner immediately precedes the peptide, or alternatively, the first concatemeric copy of the peptide. In a preferred embodiment relating to concatemeric peptides, the first peptide immediately precedes the second peptide (and so on).

For example, the amino acid sequence of a fusion polypeptide comprising the B72R leader fusion peptide and a VSDL peptide may be as follows:

The arrow denotes the location of trypsin cleavage sites.

In another example, the amino acid sequence of a fusion polypeptide comprising the B72R leader fusion peptide and three concatemeric VSDL peptides may be as follows:

The person skilled in the art will appreciate that more concatameric VSDL peptides may be included in the fusion polypeptide as described herein. For example, the amino acid sequence of a fusion polypeptide comprising the B72R leader fusion peptide and ten concatemeric VSDL peptides would be as follows: and so on.

Accordingly, in an embodiment, a single peptide cleavage site may be located between the leader fusion partner and the following peptide (or, in the case of a fusion polypeptide comprising concatemeric peptides, between the leader fusion partner and the first peptide as well as between each of the concatemeric copies of the peptide). However, it is to be understood that it may be possible for a fusion polypeptide to contain short linker sequences between each of the concatemeric copies of the peptide, wherein the linker sequences are flanked by peptide cleavage sites, so that the intact peptide is released from the fusion polypeptide upon cleavage with a cleaving agent in accordance with the present invention.

Alternatively or additionally, it is to be understood that it may be possible for a fusion polypeptide to contain short linker sequences between each of the peptides, wherein following cleavage with a cleaving agent, the linker sequences remain attached to one terminus of the peptide such that the peptide is released from the fusion polypeptide in a linker-extended form, and that the linker sequences can then optionally be cleaved from the peptide by a second cleaving agent using an alternative peptide cleavage site, such that the peptide is released from the linker sequences.

Using expression constructs encoding fusion polypeptides of the present invention and *E. coli* as a recombinant host cell, the present inventors were able to obtain suitably high levels of expression of soluble fusion polypeptides, including fusion polypeptides with one VSDL peptide and three, six, eight and ten concatemeric copies of the VSDL peptide in accordance with an embodiment of the present invention. Moreover, the present inventors have demonstrated that the obtained fusion polypeptide could be completely digested to yield suitably high concentrations of the released VSDL peptide.

In an embodiment of the present invention, the released peptide is purified. Purification of the peptide can advantageously reduce contaminants (for example, cellular components including host cell protein, nucleic acid, and endotoxin) to acceptable levels suitable for therapeutic use. The peptide can be purified using any suitable technique known to the person skilled in the art. In an embodiment, the fusion polypeptide is purified prior to cleavage with the cleaving agent. In an embodiment, the peptide is purified following cleavage of the fusion polypeptide with the cleaving agent. It is advantageous if the purification method does not require the peptide to be eluted in high concentrations of toxic, volatile or flammable solvents (eg methanol), if the peptide is to be used clinically.

In an embodiment, the method further comprises a step of purifying the released peptide from the cleaved fusion polypeptide. The person skilled in the art will be aware of a large range of purification techniques may be suitable for purifying the released peptide, for example, ion exchange techniques and other separation techniques. Ion exchange techniques that may be suitable for purifying the peptide include commercially available resins and membranes such as ChromaSorb membranes (Merck-Millipore, Billerica, MA, United States of America), Sartobind membranes (Sartorious AG, Goettingen, Germany), Mustang Membranes (Pall Corporation, Port Washington, NY, United States of America), CIM QA Monolith membranes (BLA Separations GmbH, Villach, Austria), reversed phase resins, CaptoMMC (a mixed mode resin; GE Healthcare, Waukesha, WI, United States of America), hydroxyapatite (a mixed mode resin: BioRad Laboratories, Inc, Hercules CA, United States of America), Q and DEAE monoliths (anion-exchange resins; BIA Separations), Macroprep HQ and DEAE (anion-exchange resins; BioRad), POROS HQ, PI and D (anion-exchange resins; PerSeptive Biosystems Inc, Framingham MA, United States of America), Nuvia Q (an anion-exchange resin; BioRad), UNOsphere Q (anion-exchange resin; BioRad), Q Sepharose FF and BB (anion-exchange resins; GE Healthcare), SP Sepharose FF (cation-exchange resins; GE Healthcare), Fractogel TMAE (an anion-exchange resin; Merck-Millipore), MEP Hypercel (a mixed mode resin; Pall Corporation), etc. Techniques that utilise non-ion exchange separation techniques that may be suitable for purifying the released peptide include Blue Sepharose (a pseudo-affinity resin; GE Healthcare), a number of resins sourced from Phenomenex or Vydac, etc.

In the case of the VSDL peptide (or a variant or modified peptide thereof), in one embodiment, the released VSDL peptide (or a variant or modified peptide thereof) is purified from the digested fusion polypeptide and other host cell contaminants using reversed phase chromatography. For example, the reversed phase chromatography may utilise an insoluble polystyrene divinylbenzene polymeric resin such as the Amberchrom CG300C or Amberchrom XT columns (Rohm and Haas). In some embodiments, the released VSDL peptide (or a variant or modified peptide thereof) can be eluted from such resins using 40 to 20% isopropanol (v/v). Preferably, the said elution occurs in the presence of acetic acid. For example, the VSDL peptide (or a variant or modified peptide thereof) may be eluted in 30% isopropanol in the presence of 0.5% acetic acid. In an alternative embodiment, the released VSDL peptide (or a variant or modified peptide thereof) can be eluted from such resins using 10 to 20% isopropanol. Preferably, the said elution occurs at a slightly basic pH. For example, the VSDL peptide (or a variant or modified peptide thereof) may be eluted in 15% isopropanol buffered to pH 8, eg in the presence of 25 mM Tris pH 8. Advantageously, such low concentrations of isopropanol are considered to be acceptable in large scale recombinant protein production.

In another embodiment, the released VSDL peptide (or a variant or modified peptide thereof) is purified from the digested fusion polypeptide using anion-exchange chromatography. For example, the anion-exchange chromatography may be strong anion-exchange chromatography utilising a cross-linked polymethacrylate resin such as the Fractogel TMAE columns (Merck). In some embodiments, the VSDL peptide (or a variant or modified peptide thereof) can be eluted at high purity from such resins using sodium acetate. Preferably, the said elution occurs at an acidic pH (eg 10 mM Na acetate at pH 5.0, pH 4.0 or pH 3.5). In other embodiments, the released VSDL peptide (or a variant or modified peptide thereof) can be eluted at high purity from such resins using a low concentration of hydrochloric acid (eg 10 mM HCl).

The released VSDL peptide (or a variant or modified peptide thereof) may be purified from the digested fusion polypeptide using reversed phase chromatography. For example, the reversed phase chromatography may utilise an insoluble polystyrene divinylbenzene polymeric resin such as the Amberchrom CG300C or Amberchrom XT columns (Rohm and Haas). The VSDL peptide (or a variant or modified peptide thereof) can be eluted from such columns in high yield using 10 to 20% isopropanol. Preferably, the said elution occurs at a slightly basic pH. For example, the VSDL peptide (or a variant or modified peptide thereof) may be eluted in 15% isopropanol buffered to pH8, eg in the presence of 25 mM Tris pH 8.

Additionally or alternatively, the released VSDL peptide (or a variant or modified peptide thereof) can be purified using Chromasorb membranes and eluted with 10 mM hydrochloric acid.

The released VSDL peptide (or a variant or modified peptide thereof) can be initially purified from the digested fusion polypeptide using reversed phase chromatography, for example using an insoluble polystyrene divinylbenzene polymeric resin, and then may be further purified using strong anion-exchange chromatography (for example, using a cross-linked polymethacrylate resin).

The peptide may be eluted in organic solvents selected from isopropanol, butanol and acetonitrile.

The person skilled in the art will appreciate that other purification methods may be suitable for purifying the VSDL peptide (or a variant or modified peptide thereof) following cleavage of the fusion polypeptide with the cleaving agent.

In an embodiment, the purified peptide may be lyophilised. The lyophilised peptide may then be reconstituted.

The purified peptide may be formulated with a pharmaceutical or veterinary acceptable excipient or carrier.

The present invention provides a method of producing the recombinant vessel dilator (VSDL) peptide (or a variant or modified peptide thereof) comprising the following steps:
expressing a fusion polypeptide comprising a leader fusion partner and at least one VSDL peptide (or a variant or modified peptide thereof) wherein said peptide is flanked by one or more trypsin cleavage sites;
cleaving the fusion polypeptide with trypsin or a trypsin-like enzyme;
adjusting the pH of the cleaved fusion polypeptide to below 5;
purifying the peptide from the digested fusion polypeptide using anion exchange technique(s).

It is to be understood that each of the VSDL peptide (or a variant or modified peptide thereof), fusion polypeptide, leader fusion partner, trypsin cleavage sites, trypsin or trypsin-like enzymes, etc may be embodied as described above for the first aspect of the invention. For example, the fusion polypeptide may comprise concatemeric copies of the VSDL peptide (or a variant or modified peptide thereof); and/or the VSDL peptide (or a variant or modified peptide thereof) may have the sequences as embodied above; etc.

In a second aspect, the present invention provides an expression construct comprising a nucleotide sequence encoding a fusion polypeptide as defined in the first aspect of the present invention.

The expression construct comprises a nucleotide sequence encoding a leader fusion partner having a C-terminal trypsin cleavage site operably fused to at least one nucleotide sequence encoding two to twenty concatemeric copies of VSDL peptide (or a variant or modified peptide thereof) having a C-terminal trypsin cleavage site.

As used herein, the term "operably fused" will be understood by the person skilled in the art to mean that the nucleotide sequence encodes the entire fusion polypeptide in the same reading frame, in the absence of any stop codons, such that each of the peptides of the fusion polypeptide are translated.

The nucleotide sequence encoding a leader fusion partner having a C-terminal trypsin cleavage site may be operably fused to one nucleotide sequence encoding a VSDL peptide (or a variant or modified peptide thereof) having a C-terminal trypsin cleavage site. In this aspect of the invention, the nucleotide sequence encoding a leader fusion peptide having a C-terminal trypsin cleavage site is operably fused to any number of concatemeric nucleotide sequences, each encoding a VSDL peptide (or a variant or modified peptide thereof) having a C-terminal trypsin cleavage site. The nucleotide sequence encoding a leader fusion peptide having a C-terminal trypsin cleavage site is operably fused to two to twenty (or more) concatemeric nucleotide sequences each encoding a VSDL peptide (or a variant or modified peptide thereof) having a C-terminal trypsin cleavage site. That is, the nucleotide sequence may preferably encode a fusion polypeptide comprising a leader fusion peptide having a C-terminal trypsin cleavage site which is operably fused to two, three, four, five, six, seven, eight, nine, ten, eleven, twelve, thirteen, fourteen, fifteen, sixteen, seventeen, eighteen, nineteen or twenty concatemeric copies of the VSDL peptide (or a variant or modified peptide thereof) having a C-terminal trypsin cleavage site.

Preferably, the C-terminal trypsin cleavage sites are C-terminal arginine residues.

In an embodiment, the nucleotide sequence encodes a fusion polypeptide comprising or consisting of an amino acid sequence selected from the group consisting of SEQ ID NO: 11, SEQ ID NO: 12 and SEQ ID NO: 13.

The expression construct may be located within an expression plasmid as will be well known to the person skilled in the art.

In a third aspect, the present invention provides a host cell for recombinantly expressing a peptide comprising the expression construct of the second aspect. A wide range of expression systems that can produce recombinant peptides are known to the person skilled in the art. Further, the host cells of the present invention may be any suitable host cell as will be appreciated by the person skilled in the art. The host cell may include cells of bacteria (eg *Escherichia coli, Streptomyces sp., Bacillus sp., Pseudomonas sp., Staphylococcus sp.* (eg. *S. typhimurium* etc), etc), fungal cells such as yeast cells (eg *Saccharomyces cerevisiae, Pichia pastoris,* etc), molds or filamentous fungi (eg *Aspergillus sp*.), insect cells (such as Drosophila S2 and *Spodoptera frugiperda* (such as Spodoptera Sf9 cells), and including baculovirus-assisted systems), mammalian cells (numerous cell lines are suitable including Chinese Hampster Ovary (CHO) cells, monkey kidney (COS) cells, human embryonic kidney (HEK) cells, baby hampster kidney (BHK) cells, murine melanoma cells, etc), and plant cells. Recombinant peptides can alternatively be expressed in transgenic plants and animals, and accordingly, the host cell may be a suitable plant or animal cell. Preferably, the host cell is an *E. coli* cell.

In a further aspect, the present invention provides a recombinant fusion polypeptide comprising a leader fusion partner linked to two to twenty concatemeric copies of a VSDL peptide (two to twenty concatemeric copies of the peptide or more) having a C-terminal trypsin cleavage site. The leader fusion partner has a C-terminal trypsin cleavage site. Preferably, the peptide has no internal trypsin cleavage sites. The VSDL peptide comprises or consists of the amino acid sequence according to SEQ ID NO: 2.

The present disclosure provides a recombinant fusion polypeptide comprising or consisting of concatemeric copies of a peptide (eg two to twenty concatemeric copies of the peptide or more) having a C-terminal trypsin cleavage site. The polypeptide may comprise three to ten concatemeric copies of the peptide. The peptide may have no internal trypsin cleavage sites. The peptide may be a VSDL peptide or a variant or modified peptide thereof, or a VSDL peptide comprising or consisting of the amino acid sequence according to SEQ ID NO: 2.

The invention is hereinafter described by reference to the following non-limiting example and accompanying figures.

### EXAMPLES

### Example 1 Recombinant production of VSDL in E. coli

The amino acid sequence of human VSDL (designated VSDL in the Examples) is:

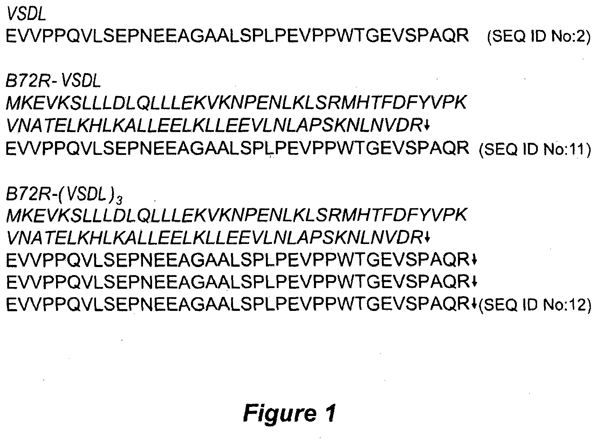

In this example, the recombinant expression of VSDL in *E. coli* was investigated using a fusion polypeptide approach in a strategy attempting to exploit the absence of internal arginine (R) and lysine (K) residues in the peptide sequence, as well as the presence of a C-terminal arginine (R) that conveniently provides a cleavage site for trypsin (Figure 1).

### Generation of expression constructs

Initially, two fusion polypeptides were designed:
(i) a single VSDL sequence fused with a 73 amino acid fusion partner designated B72R (designated B72R-VSDL and also referred to as a "1-mer" herein), and
(ii) a triple VSDL repeat fused with B72R (designated B72R-(VSDL)₃ and also referred to as a "3-mer" herein).The amino acid sequence for these fusion polypeptides is shown in Figure 1.

A second round of gene fusions were then generated in which the penultimate aspartic acid residue (D) of the fusion partner was replaced with a glutamine residue (Q) (designated B72(Q)R), and the encoded fusion polypetides comprised three, six, eight and ten tandem copies of the VSDL peptide sequence. It was anticipated that the substitution of the aspartic acid residue might result in improved cleavage at the fusion partner/VSDL junction.

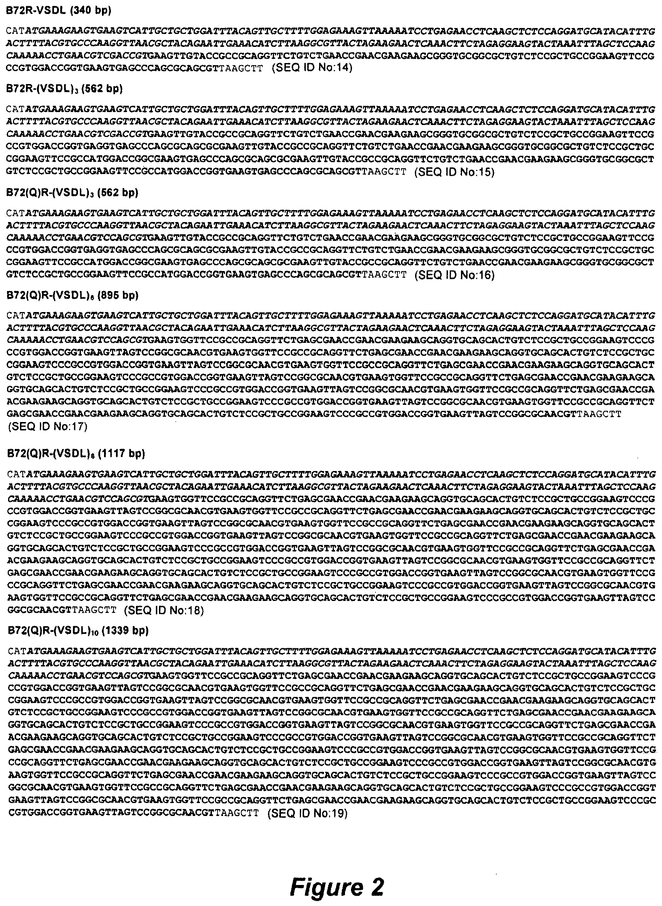

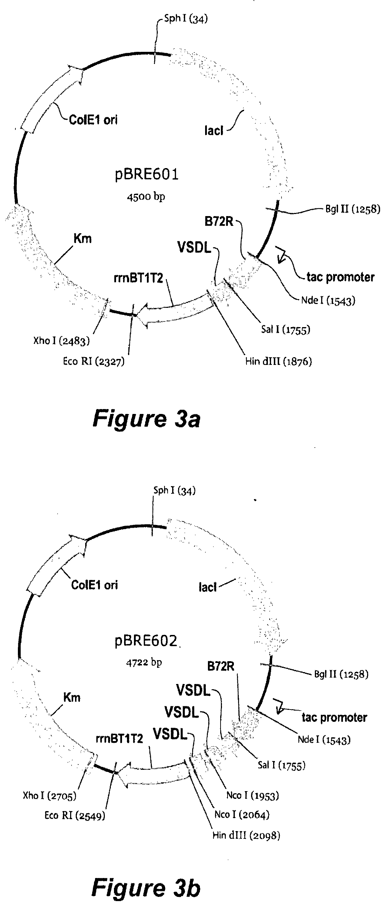

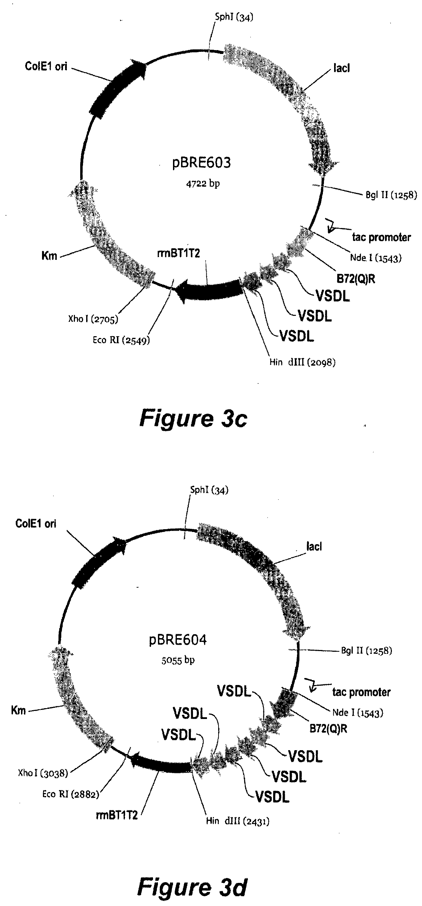

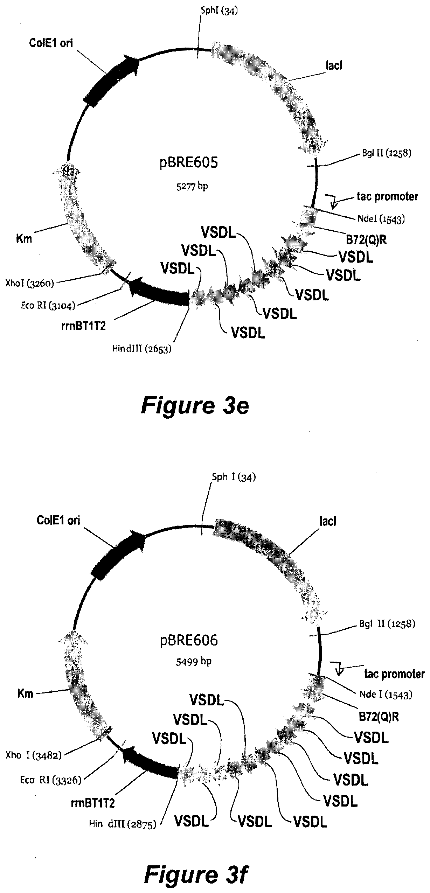

VSDL fusion polypeptide genes were obtained as pUC57 vector subclones (GenScript USA Inc, Piscataway NJ, United States of America). The nucleotide sequences of the gene fusions are shown in Figure 2. The VSDL gene fusions were excised from the respective pUC57 subclones by digestion with the restriction enzymes *NdeI* and *HindIII* (Roche Diagnostics Corporation, Indianapolis IN, United States of America). The plasmid digests were desalted and then directly ligated with a pBRE508 expression vector (Hospira Adelaide Pty Ltd, Thebarton SA, Australia) by incubation at 16°C overnight in the presence of T4 DNA ligase (Roche). Ligation reactions were used to transform XL1-Blue electrocompetent cells (Agilent Technologies Inc, Santa Clara CA, United States of America) and positive transformants selected for on VA plates containing kanamycin (30 *µ*g/mL). Plasmid DNA was prepared from individual transformants using a QIAprep Spin Miniprep Kit (Qiagen NV, Venlo, The Netherlands) according to the manufacturer's instructions. The purified plasmids were then digested with *NdeI* / *HindIII* to identify successful candidate clones. Plasmid clones exhibiting the expected banding pattern for the different VSDL gene fusions were designated pBRE601 through to pBRE606. Maps of the VSDL expression constructs are depicted in Figures 3a-f.

### Small scale expression studies

Initial expression experiments were performed at the 10 mL shake flask scale. The VSDL expression constructs, pBRE601 (1-mer) and pBRE602 (3-mer), were transformed into *E. coli* host strain BR067 (Hospira Adelaide; MM294 OmpT deletion) and transformants selected for on VA plates containing kanamycin (30 *µ*g/mL).

Overnight 3 mL cultures were established by aseptically transferring single colonies from the transformation plates into 3 mL C2-defined media cultures with kanamycin (30 *µ*g/mL). The cultures were incubated for 16 hours at 37 °C with agitation via a rotating carousel. Glycerol stocks were prepared by combining 0.875 mL of each culture with 0.125 mL 80% glycerol followed by storage at - 80 °C.

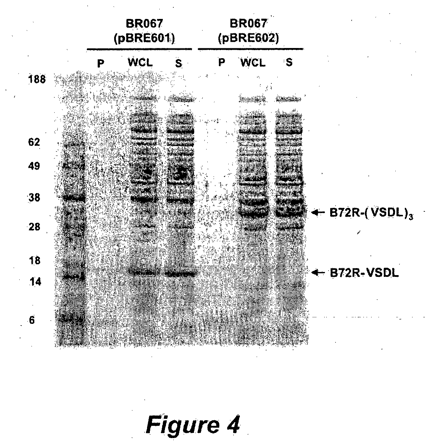

For the expression experiments, overnight cultures (1 mL) were aseptically subcultured into 10 mL C2-defined media with kanamycin (30 *µ*g/mL). These cultures were incubated at 37 °C with agitation for 2.5 hours and then induced with IPTG (0.25 mM final concentration). After 3 hours of further incubation, SDS-PAGE samples were prepared. SDS-PAGE analysis of fractionated BR067(pBRE601) and BR067(pBRE602) culture samples (Figure 4) showed high level, soluble expression of BR72R-VSDL and B72R-(VSDL)₃ fusion polypeptides.

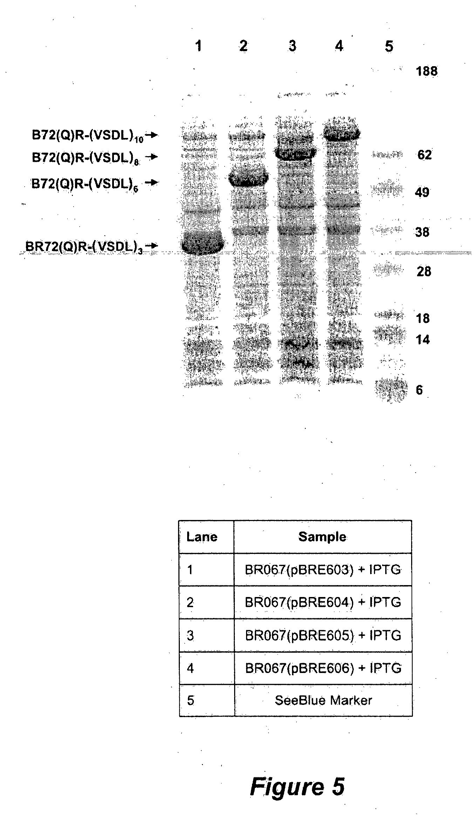

Subsequently, equivalent expression experiments were conducted with constructs encoding B72(Q)R fusion polypeptides comprising 3, 6, 8 and 10 tandem copies of VSDL. As shown in Figure 5, the cultures also demonstrated high level, soluble expression of fusion polypeptides. It was, however, noted that as the number of copies of the VSDL sequence increases so too does aberrant migration behaviour of the fusion polypeptide (Table 4). This is most likely the consequence of the high negatively charged nature of the VSDL peptide sequence.

**Table 4 Predicted and apparent molecular weight of VSDL fusion polypeptides**

| **Expression construct** | **VSDL copies** | **Predicted Mᵣ (kDa)** | **Apparent Mᵣ (kDa)** |
|---|---|---|---|
| pBRE601 | 1 | 12.7 | 17 |
| pBRE602/pBRE603 | 3 | 20.3 | 35 |
| pBRE604 | 6 | 31.9 | 53 |
| pBRE605 | 8 | 39.7 | 62 |
| pBRE606 | 10 | 47.4 | 80 |

### Fed-batch fermentations

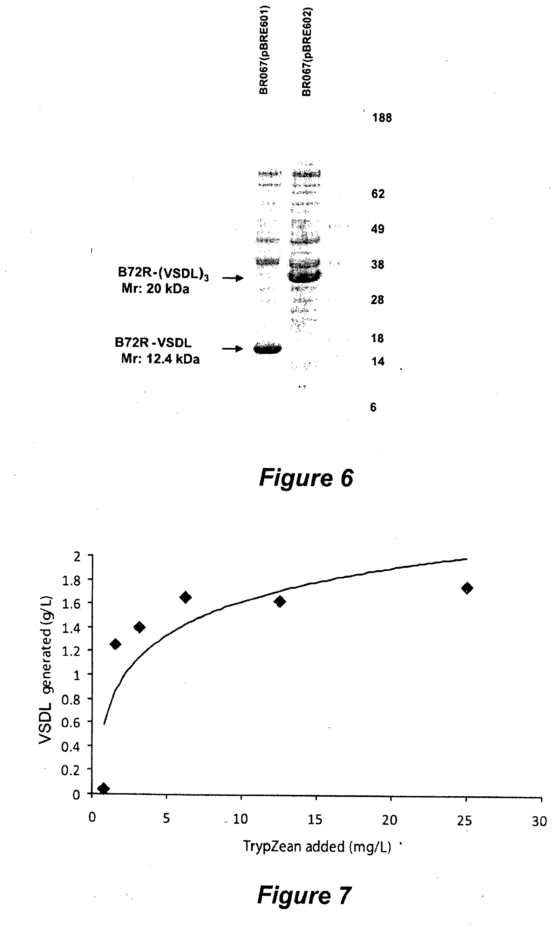

Fed-batch cultivations were carried out in 1 L reaction vessels coupled to a BioStat Qplus bioprocess control unit (Sartorius). Runs were initiated from a frozen glycerol stock of cell lines BR067(pBRE601) and BR067(pBRE602), respectively. Nutrient feeding was commenced following overnight growth at 37°C in batch mode. When cultures reached an OD600 of 40-50, they were induced by the addition of IPTG and growth continued for 5 hours. The final OD600 achieved with each cell line was ∼70 and a final biomass wet weight of ∼80 g/L. SDS-PAGE analysis of final culture samples, shown in Figure 6, demonstrated that a high level expression of BR72-VSDL and B72R-(VSDL)₃ was achieved under these conditions.

The fermentation biomass of the two cell lines was recovered by centrifugation and resuspended in 25 mM Tris HCl pH 8.0. Clarified lysates were then generated following high pressure disruption (3 passes at 920 bar) of the collected cells and centrifugation (8000 rpm for 15 minutes). Lysates were aliquoted and stored at -80°C and this material was used for cleavage and purification studies described below.

### Cleavage of fusion polypeptide

Lysates comprising the B72R-VSDL and B72R-(VSDL)₃ fusion polypeptides were thawed. Sequencing grade modified porcine trypsin (Promega V511A; Promega Corporation, Fitchburg WI, United States of America) was added at 25 *µ*g/mL and incubated at room temperature overnight. The samples were mixed 1:1 with 1.0% H₃PO₄/0.2% TFA and centrifuged to remove precipitate. The supematants were analysed by RP-HPLC on a Waters Alliance HPLC Separation Module and UV Detector (Waters Corporation, Milford MA, United States of America). Analysis was performed on a Phenomenex Jupiter C4, 250 x 2.1 mm, 5 *µ*m, 300 A column (#302) under the control of Empower 2 software (Phenomenex, Inc, Torrance CA, United States of America). Three injections of VSDL synthetic peptide (ie chemically synthesised VSDL) of 5, 10 and 20 *µ*g were used to construct a standard curve, which was used to quantify amounts of recombinant VSDL generated in the cleaved lysate.

Both fusion polypeptides eluted at a retention time of 38 min, whereas VSDL eluted at 28 min. Recombinant VSDL and synthetic VSDL retention times were comparable. The fusion polypeptides were digested under the conditions tested. Previous investigations had revealed the presence of a partially digested (N-terminally extended) variant, designated NLNVDR-VSDL, which elutes at a later retention time by RP-HPLC. The digests were analysed by a slower gradient and it was confirmed that significant amounts of this variant remained; a greater proportion of the variant being present in the 1-mer digest than in the 3-mer digest.

VSDL concentrations of 0.58 g/L and 1.49 g/L were determined by RP-HPLC in the B72R-VSDL and B72R-(VSDL)₃ digests, respectively. If the digestion had gone to completion; that is, until none of the N-terminally extended variant NLNVDR-VSDL was present, then the VSDL concentrations would be increased to 0.91 g/L and 1.76 g/L in the 1-mer and 3-mer digests, respectively.

Taking into account the relative molecular weights for VSDL and the fusion partner, it was estimated that the fusion polypeptide concentrations were 2.91 g/L and 3.04 g/L in the 1-mer and 3-mer lysates, respectively. However, during sample preparation, acidification results in a significant amount of visible precipitation and, since the concentrations of fusion polypeptide measured by RP-HPLC in the undigested lysates were estimated to be significantly less than the above quoted figures, it was considered that significant amounts of the B72R-VSDL and B72R-(VSDL)₃ fusion polypeptides may be susceptible to precipitation alongside other *E*. *coli*-derived proteins present in the crude lysates.

Examination of the amino acid sequence reveals that "authentic" VSDL (ie from either the 1-mer and 3-mer fusion polypeptides) is generated when cleavage occurs at the cleavage site of VDR↓E at the C-terminal end of the fusion partner B72R, and the cleavage site of AQR↓E at the C-terminal end of the VSDL sequence(s) (wherein ↓ indicates the location of the peptide cleavage site). In comparison, the peptide cleavage site for the N-terminally extended variant (NLNVDR-VSDL) is PSK↓N. The lower cleavage efficiency at VDR↓E is likely due to the presence of the aspartic acid residue (D) immediately preceding the arginine (R) cleavage site, which may reduce the affinity of trypsin for this site. Since the relatively small difference in physicochemical properties between VSDL and NLNVDR-VSDL would be likely to increase the difficulty of the purification to remove variant from the final product, it is was hypothesised that modification of the cleavage site at the C-terminal end of the fusion partner from VDR↓E to VQR↓E (such that the terminal and penultimate amino acid residues at the C-terminal end of the fusion partner where the same as those at the C-terminal end of the VSDL peptide(s)) would improve the overall cleavage efficiency. Moreover, increasing the number of the VSDL sequences may also decrease the prevalence of the variant whilst further increasing the yield as the mass ratio of fusion partner to fusion polypeptide declines.

The 3-mer fusion polypeptide (ie B72R-(VSDL)₃) was purified by loading 4 mL lysate on a preparative RP-HPLC column (Phenomenex Jupiter C4, 250 x 10 mm, 5 *µ*m, 300 Å). The fusion polypeptide was eluted with a 5-60% acetonitrile gradient over 60 min in the presence of 0.1% TFA. The acetonitrile and TFA were evaporated from the protein with a centrifugal freeze-drier, and the protein then reconstituted to 0.5 mg/mL in 50 mM Tris, 1 mM CaCl2 pH 8.0. A 10 mg vial of recombinant bovine TrypZean® (Sigma-Aldrich) was resuspended to 5 mg/mL in 50% glycerol/50 mM acetic acid and stored at - 20°C. TrypZean® was added to fusion polypeptide at a mass ratio of 1:120 and digestion occurred overnight at room temperature. After 10 min the samples were acidified with 1% acetic acid to terminate the digestions. The digested and undigested samples were analysed by LC-MS. The samples were also chromatographed through a Zorbax C3 column (Agilent), eluting with a gradient of acetonitrile in the presence of 1% acetic acid, coupled to a Bruker HCT*ultra* Ion-Trap mass spectrometer. The undigested sample yielded a mass of 20091.4 Da, which compares with the theoretical mass of the 3-mer fusion polypeptide (ie 20091.0 Da). The digested sample yielded a mass of 3877.8 Da, which compares with the theoretical mass of VSDL (ie 3878.3 Da). Other peptides that were detected correspond to partially digested fragments of the fusion partner. Notably, there was no evidence of VSDL concatemers or linkage to the fusion partner. The absence of the NLNVDR-VSDL variant was confirmed by RP-HPLC and indicated that the cleavage efficiency was improved by the use of TrypZean® and/or the purification of fusion polypeptide prior to digestion. Subsequent cleavage experiments with lysate showed that TrypZean® at a 1:120 (w/w) cleavage ratio did not leave residual NLNVDR-VSDL.

The requirements for TrypZean® were determined by adding various amounts to a fixed amount of the B72R-(VSDL)₃ lysate (3 g/L fusion polypeptide) and incubating overnight at room temperature. Amounts of cleaved VSDL were determined by RP-UPLC on a Shimadzu CBM-20A (Shimadzu Corporation, Kyoto, Japan) using a Restek Pinnacle DBC8, 100 x 2.1 mm, and 1.9 *µ*m column. Three injections of synthetic VSDL peptide of 5, 10 and 15 *µ*g were used to construct a standard curve, which was used to quantify recombinant VSDL. Above 6 mg/L TrypZean®, the equivalent of a 1:480 (w/w) TrypZean® to fusion polypeptide ratio, the generated recombinant VSDL begins to plateau (Figure 7).

### Purification

The aim of this experiment was to demonstrate a purification process that: (1) gives a purified VSDL yield of greater than 0.25 g per L ferment, (2) demonstrates a purity profile comparable to the synthetic VSDL peptide, (3) reduces endotoxin to low levels, (4) formulates the VSDL in 10 mM HCl, (5) uses organic solvent amounts compatible with large-scale production, (6) uses low pressure chromatographic steps, and (7) is scalable.

### Initial process

In previous work, a purification process had been developed that utilised the steps of (1) trypsin cleavage, (2) precipitation of host cell impurities by acidification, (3) reversed phase separation with acid/methanol, (4) pH adjustment to 8, and (5) anion-exchange separation with NaCl. This process was repeated by loading 15 mL digested 3-mer lysate on a 1 mL Amberchrom XT30 column (Rohm and Haas) followed by a 1 mL Q Sepharose HP column (GE Healthcare, Little Chalfont, Buckinghamshire, United Kingdom). Approximately 10 mg of VSDL was purified with the Q column with a yield of 1.23 g per L ferment. The elution position and impurity profile of the purified VSDL compared favourably by RP-HPLC with the synthetic VSDL peptide and the N-terminally extended NLNVDR-VSDL variant was absent. However, this purification process utilised 100% methanol to elute bound VSDL from the column, and the volatility, flammability and toxicity of methanol therefore means that this purification process may be undesirable for large-scale production.

### Reduction of Organic Solvent Usage

The potential for reducing organic solvent usage during purification with Amberchrom reversed phase resins was also investigated.

Digested 3-mer lysate was loaded on Amberchrom CG300C (Rohm and Haas) and eluted with a methanol gradient. As for XT30, 100% methanol was required to elute VSDL. However, in subsequent experiments, the methanol was replaced by isopropanol and it was found that VSDL could be eluted with 30% isopropanol at high purity in the presence of 0.5% acetic acid. Nucleic acid contaminants could be washed from the column with 15% isopropanol prior to VSDL elution. Lysate without pH adjustment was also loaded onto the column and isopropanol elution performed in the presence of 25 mM Tris pH 8 rather than 0.5% acetic acid. Unexpectedly, it was found that VSDL eluted at 15% isopropanol for the higher pH. Notably, the flammability of 15% isopropanol is significantly less than 30% isopropanol, and indeed similar to that for 20% ethanol (nb. the flash points are identical - 35°C), which is commonly used in large scale recombinant protein production.

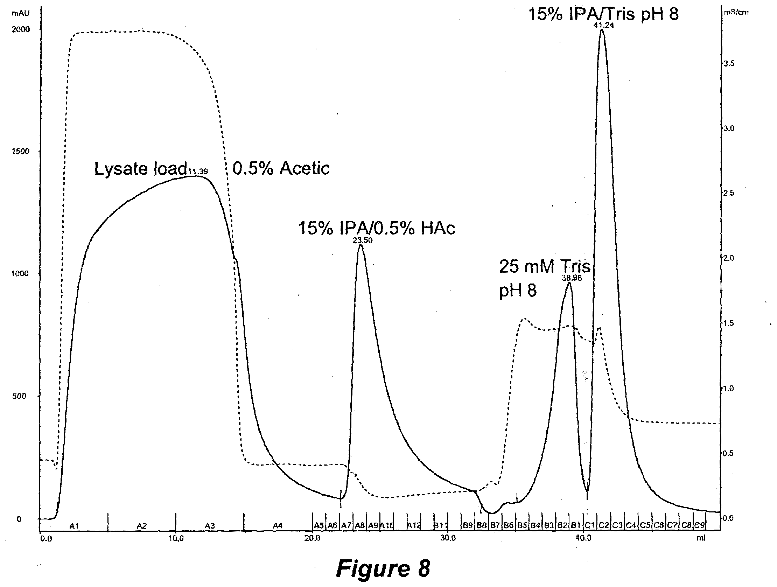

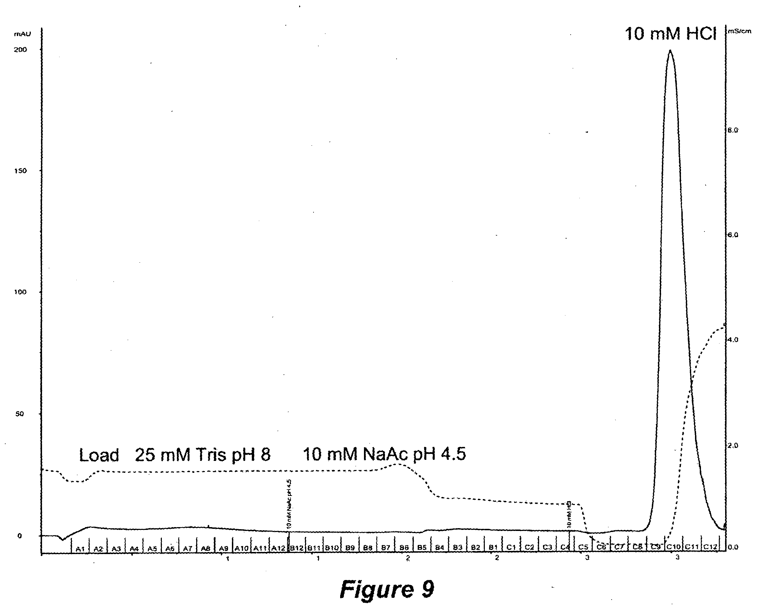

Lysate was digested overnight at room temperature with a 1:120 mass ratio of TrypZean®. The pH was adjusted to 4.4 with 1M acetic acid on the following day to precipitate host cell impurities. The precipitate was then settled with centrifugation and 13 mL of supernatant loaded on a 1 mL Amberchrom CG300C column, which had been pre-equilibrated with 0.5% acetic acid. The column was washed with 0.5% acetic acid, 15% isopropanol/0.5% acetic acid and 25 mM Tris pH 8 (Figure 8). A large peak was eluted with 15% isopropanol/25 mM Tris pH 8. The peptide content in the peak was 18.5 mg, which translates to a step recovery of 81%. Subsequently, a 1 mL Fractogel TMAE column was pre-equilibrated with 25 mM Tris pH 8 and loaded with 6.9 mg of this material. The column was then washed with 25 mM Tris pH 8 and 1.0 mM sodium acetate pH 4.5 (Figure 9). A large peak was eluted with 10 mM HCl, with a peptide content of 5.8 mg, which demonstrates a step recovery of 84%. The purified yield was 1.37 g per L ferment.

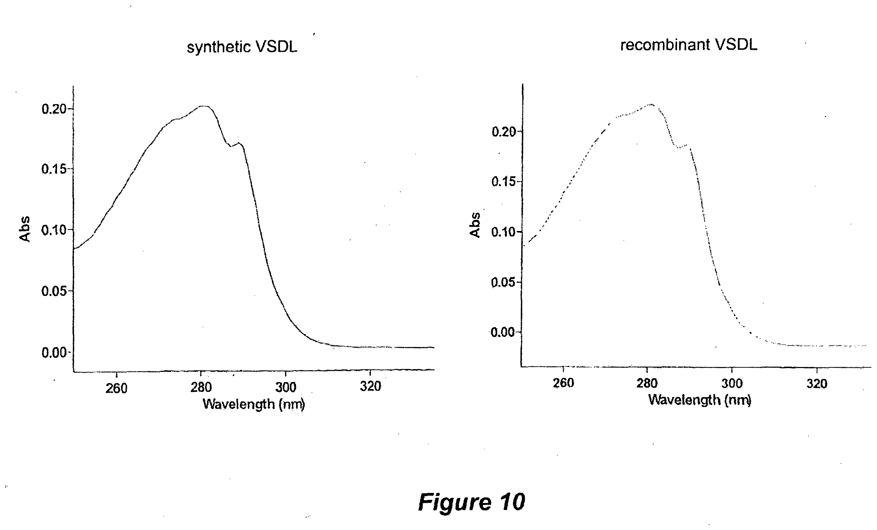

The binding capacities of Amberchrom CG300C and Fractogel TMAE for VSDL were ∼20 mg/mL and ∼7 mg/mL, respectively. The large peak was compared with the synthetic VSDL peptide by RP-UPLC analysis and purities of 97.0% and 93.7% were measured, respectively. There were no new significant impurity peaks for VSDL relative to the synthetic VSDL peptide. Further, the synthetic VSDL peptide and recombinant VSDL UV profiles were almost identical (Figure 10). Moreover, the endotoxin content of a Fractogel VSDL peak, purified in a similar fashion, was low (5 EU/mg).

### Conclusions

The results shown in the example demonstrate the development of a cell line that expresses VSDL as a stable fusion polypeptide at good yield, and a purification process of the VSDL in a manner compatible with large scale production.

This involved the design of a fusion polypeptide to exploit the C-terminal arginine residue (R) of VSDL as a cleavage site for trypsin, which is a well characterised enzyme that is available as a non-animal-derived recombinant protein, TrypZean®. Initially, two fusion polypeptides were designed: a single VSDL sequence fused with B72R (1-mer) and a triple VSDL repeat sequence fused with B72R (3-mer), which were expressed in a soluble form, thereby avoiding the need to purify expressed protein from isolated inclusion bodies. Further work established that VSDL could also be expressed at high levels as a 6-mer, 8-mer and 10-mer.

Preliminary trypsin cleavage studies using a laboratory-grade porcine trypsin source demonstrated that the fusion polypeptides can be completely digested to yield VSDL peptide, which could be subsequently recovered with a purification process comprising the steps of: (1) overnight digestion with 1:120 TrypZean®: fusion polypeptide (w/w), (2) pH adjustment to 4.4, (3) centrifugation, (4) Amberchrom CG300C reversed phase chromatography, and (5) Fractogel TMAE anion-exchange chromatography. The Fractogel material was compared with a synthetic VSDL preparation by RP-UPLC analysis and purities of 97.0% and 93.7% were measured, respectively. Importantly, there were no new significant impurity peaks for the recombinant VSDL (ie relative to the synthetic VSDL peptide) and the UV profiles of the recombinant and synthetic VSDL were found to be almost identical. Further, the endotoxin content of the purified recombinant VSDL was estimated as 5 EU/mg.

Therefore, it was found that the purification process: (1) gives a purified recombinant VSDL yield of 1.2-1.4 g per L ferment, (2) demonstrates a purity profile comparable to a synthetic VSDL preparation, (3) reduces endotoxin to low levels, (4) formulates the VSDL in 10 mM HCl, (5) uses organic solvent amounts that are compatible with large scale production, (6) uses low pressure chromatographic steps, and (7) is scalable.

### Example 2 Recombinant production of VSDL 10mer in E. coli

Lysate was generated from the fermentation of the BR067 *E. coli* strain harbouring the pBRE606 expression construct described in Example 1, which encodes the B72R-(VSDL)₁₀ fusion polypeptide having 10 concatemeric copies of the VSDL peptide (ie the 10mer), as described in the "Fed-batch fermentations" section of Example 1.

275 mL of the 10mer lysate was thawed, and the pH was adjusted to pH 8.0 with 1M NaOH. TrypZean® (as described in Example 1) was added to fusion polypeptide at a mass ratio of 1:120 and digestion occurred for two hours at room temperature. The pH of the digested lysate was adjusted to pH 4.4 with 1 M acetic acid to precipitate impurities, and then depth filtered with Millistak+ Pod Labscale D0HC filter (MD0HC027H2) 0.027 m².

The filtrate was purified by directly loading onto a column of reversed-phase Amberchrom CG300C resin. The column was washed with approximately 15 column volumes 0.5% acetic acid, then approximately 1.0 column volumes 0.5% acetic acid/15% isopropanol; then approximately 15 column volumes 25 mM Tris pH 8. The column was eluted with 25 mM Tris/15% isopropanol until the UV absorbance reached 10 % of the main peak. The eluate was then loaded onto Chromasorb membranes (Merck-Millipore), washed with 10 mM sodium acetate pH 5 until UV baseline was attained, and then washed with 10 mM sodium acetate/50 mM sodium chloride pH 5. The membranes were then eluted with 10 mM sodium acetate/200 mM sodium chloride until UV baseline was attained. The Chromasorb eluate was then loaded back onto the filters, washed with 10 mM sodium acetate pH 5 until UV baseline was attained, and then eluted with 10 mM HCl until the UV baseline was attained. The VSDL peptide was recovered with a yield of approximately 1 mg/ml lysate.

The Chromasorb eluate was aliquoted into a number of tubes/vials, and frozen at -70°C for approximately 30 min, and then transferred to a Labconco rotary-freeze-drier for lyophilisation as follows. The samples were frozen at -25 deg C in the Labconco for 30 min, and then a vacuum was applied to the samples. Primary drying occurred at -15°C for 24 hr. Secondary drying occurred overnight at +17°C. The lyophilised tubes/vials were then stored in a -20°C freezer.

Lyophilisate was reconstituted in 0.9% (w/v) NaCl and analysed by a panel of assays using standard techniques (Table 5).

**Table 5 Analysis of reconstituted lyophilised recombinant VSDL**

| Assay | Result |
|---|---|
| Purity (SEC) | 99.2 % |
| HMW impurity (SEC) | 0.1 % |
| Purity (RP-UPLC) | 98.8 % |
| Highest single impurity (RP-UPLC) | 0.3 % |
| Endotoxin | < 0.6 EU/mg |
| Host cell protein (ELISA) | < 12 ng/mg |
| DNA | < 2600 pg/mg |
| N-terminal sequencing | Correct sequence |

| | |
|---|---|
| *nb SEC is an acronym for size exclusion chromatography, HMW is an acronym for high molecular weight, RP-UPLC is an acronym for reversed phase ultra-high pressure liquid chromatography | |

### Example 3 Recombinant production of VSDL 3mer in E. coli

VSDL lysate was acquired as per Example 2, except that the of the BR067 *E. coli* strain used harboured pBRE602 expression construct described in Example 1, which encodes the B72R-(VSDL)₃ fusion polypeptide having 3 concatemetric copies of the VSDL peptide (ie the 3mer).

B72R-(VSDL)₃ lysate (175 mL) was thawed to reach a temperature between 15 and 25°C. The pH of the lysate was adjusted to 8.0 ± 0.1 with 1M NaOH. Reconstituted TrypZean was added at 25 *µ*g/mL lysate. The digest was gently mixed for 5 min, then was kept stationary overnight at room temperature. The pH of the digest was adjusted to 4.4 ± 0.1 with 1M acetic acid. The solution was gently agitated using a magnetic stirrer.

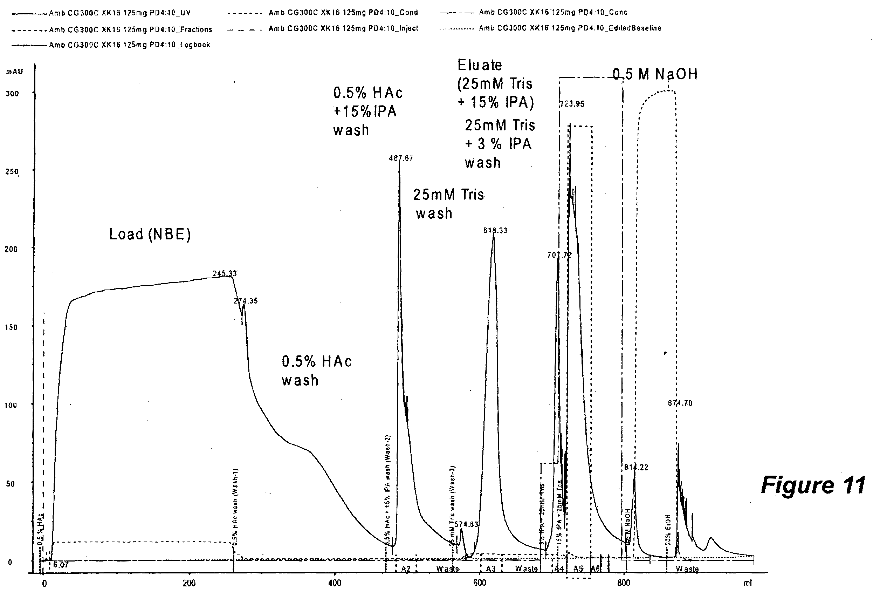

The lysate was then depth filtered through a Millistak+ Pod Labscale D0HC filter (MD0HC027H2) 0.027 m² at 4.2 mL/min, and then directly loaded onto a Amberchrom CG300C Column (16 mm ID column to a bed height of 7.0 cm. The column was washed with 0.5% acetic acid at 4.2 mL/min for approximately 50 min, then 0.5% acetic acid/15% isopropanol at 4.2 mL/min for approximately 25 min, then 25 mM Tris at 4.2 mL/min for approximately 35 min, and then 25 mM Tris/3 % isopropanol at 4.2 mL/min. When the UV absorbance starts declining from the maximum, the wash was switched to 25 mM Tris/ 15 % isopropanol. The column was eluted with 25 mM Tris/15% isopropanol at 4.2 mL/min until UV absorbance reached 10 % of the main peak. The separation of VSDL from the digested lysate is shown in Figure 11.

### Example 4 Analysis of New Construct Ferments with B72QR as leader fusion partner

Substitution of the penultimate aspartic acid residue of the B72R fusion partner with a glutamine residue was investigated for enhanced trypsin cleavage at the C-terminal arginine residue of the leader peptide. The leader fusion peptide with said substitution is referred to as B72(Q)R herein (refer to SEQ ID NO: 10). New VSDL 6-mer, 8-mer and 10-mer constructs were made that were fused to the B72(Q)R leader peptide using similar techniques as described in Example 1. The resultant constructs were transformed into *E. coli* strain BR067 and screened as described in Example 1.

The B72R-(VSDL)₃, B72QR-(VSDL)₆, B72QR-(VSDL)₈, and B72QR-(VSDL)₁₀ strains were cultured as described in Example 1. Cell lysates were incubated overnight with TypZean using a 1:120 molar ratio at room temperature. The VSDL peptide was quantified by RP-UPLC as described in Example 1. High concentrations of VSDL (2.5 mg/ml) were recorded for the VSDL 8mer and 10mer with B72QR as the leader fusion partner as shown in Table 6.

**Table 6 RP-UPLC Analysis of B72QR 6mer, 8mer or 10mer constructs compared to the B72R 3mer construct**

| **Number of VSDL copies** | **VSDL Conc** (**mg**/**mL**) |
|---|---|
| 3 | 1.96 |
| 6 | 1.53 |
| 8 | 2.54 |
| 10 | 2.54 |

### Prophetic Example I Recombinant production of GLP

The nucleotide sequence encoding native human glucagon-like peptide (GLP, derived from amino acids 98 to 127 of human glucagon preproprotein, which has NCBI Reference NP_002045) is modified to remove two internal lysine (K) residues from the corresponding amino acid sequence, such that the lysine residues are converted to glutamine (Q) residues. The native amino acid sequence naturally has a C-terminal arginine (R) residue. Expression constructs are created that encode an N-terminal His tag followed by an arginine residue (to provide a trypsin cleavage site) that is operably linked to either three or eight concatemeric copies of the modified GLP nucleotide sequence within a standard *E. coli* expression vector. The expression vectors are transformed into *E. coli* and transformants are selected and then cultured as described in Example 1.

The lysates are cleaved by overnight incubation with Trypzean using a 1:120 molar ratio. Separation of the recombinant GLP from the fusion peptide can be assessed as described in Examples 1 to 4. Optimal purification of GLP can be determined using standard techniques.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### REFERENCES

De Palo EF et al., Clin Chem 46:843-847 (2000)
Franz M et al., Kidney Int 58:374-378 (2000)
Franz M et al., Kidney Int 59:1928-1934 (2001)
Hunter EFM et al., Scan J Clin Lab Invest 58:205-216 (1998)
Kirshenbaum K et al., Curr Opin Struct Biol 9:530- 535 (1999)
Skelton WP 4th et al., Anticancer Res (2) 395-402 (2011) (Abstract)
Vesely DL et al., Biochem Biophys Res Commun 148:1540-1548 (1987)
Vesely DL et al., Proc Soc Exp Biol Med 192:230-235 (1989)
Vesely DL, Am. J. Physiology 285:F167-F177 (2003)
Winters CJ, Circulation 80:438-449 (1989)

## Claims

1. A method of producing a recombinant vessel dilator (VSDL) peptide, the method comprising the following steps:
expressing a fusion polypeptide comprising concatemeric copies of the VSDL peptide wherein each concatemeric copy of the VSDL peptide is flanked by an N-terminally located peptide cleavage site and a C-terminally located peptide cleavage site, wherein said peptide cleavage sites are otherwise absent from the VSDL peptide; and
cleaving the fusion polypeptide at the peptide cleavage site(s) with a cleaving agent(s) that cleaves at the peptide cleavage site(s);
wherein the step of cleaving the fusion polypeptide releases said peptide from the fusion polypeptide;
**characterised in that**:
the VSDL peptide consists of a native peptide sequence comprising a C terminal arginine residue;
the C-terminally located peptide cleavage site of each concatemeric copy of the VSDL peptide consists of the C terminal arginine residue; and
the C terminal arginine residue of each VSDL peptide except the last VSDL peptide in the fusion polypeptide additionally serves as the N-terminally located peptide cleavage site of a following VSDL peptide, wherein VSDL peptide comprises or consists of the amino acid sequence according to SEQ ID NO: 2.

2. The method of claim 1, wherein the peptide cleavage sites are trypsin cleavage sites, and the cleaving agent is trypsin or a trypsin-like enzyme.

3. The method of either of claims 1 or 2, further comprising the steps of:
adjusting the pH of the cleaved fusion polypeptide to below 5; and
purifying the peptide from the digested fusion polypeptide using at least one anion exchange technique.

4. The method of any one of claims 1 to 3, further comprising spray or freeze drying the peptide.

5. The method of any one of claims 1 to 4, wherein the fusion polypeptide comprises from two to twenty concatemeric copies of the peptide.

6. The method of any one of claims 1 to 4, wherein the fusion polypeptide comprises three concatemeric copies of the peptide.

7. The method of any one of claims 1 to 4, wherein the fusion polypeptide comprises ten concatemeric copies of the peptide.

8. The method of any one of claims 1 to 7, wherein the fusion polypeptide comprises a leader fusion partner having a C-terminal peptide cleavage site.

9. The method of claim 8, wherein the leader fusion partner comprises a C-terminal arginine or lysine residue.

10. An expression construct comprising a nucleotide sequence encoding a leader fusion partner operably fused to at least one nucleotide sequence encoding two to twenty concatemeric copies of a VSDL peptide, wherein the leader fusion partner has a C-terminal trypsin cleavage site;
**characterised in that**:
the VSDL peptide consists of a native peptide sequence comprising a C terminal arginine residue;
a C-terminally located peptide cleavage site of each concatemeric copy of the VSDL peptide consists of the C terminal arginine residue;
and the C terminal arginine residue of each VSDL peptide except the last VSDL peptide in the fusion polypeptide additionally serves as the N-terminally located peptide cleavage site of a following VSDL peptide, wherein the VSDL peptide comprises or consists of an amino acid sequence according to SEQ ID NO: 2.

11. A host cell for recombinantly expressing a peptide, wherein the host cell comprises the expression construct of claim 10.

12. A recombinant fusion polypeptide comprising a leader fusion partner linked to two to twenty concatemeric copies of a VSDL peptide, wherein the leader fusion partner has a C-terminal trypsin cleavage site;
**characterised in that**:
the VSDL peptide consists of a native peptide sequence comprising a C terminal arginine residue;
a C-terminally located peptide cleavage site of each concatemeric copy of the VSDL peptide consists of the C terminal arginine residue;
and the C terminal arginine residue of each VSDL peptide except the last VSDL peptide in the fusion polypeptide additionally serves as the N-terminally located peptide cleavage site of a following VSDL peptide, wherein the VSDL peptide comprises or consists of an amino acid sequence according to SEQ ID NO: 2.

## Patentansprüche

1. Verfahren zum Herstellen eines rekombinanten Gefäßdilatator(*vessel dilator-*VSDL)peptids, wobei das Verfahren folgende Schritte umfasst:
Exprimieren eines Fusionspolypeptids, konkatemere Kopien des VSDL-Peptids umfassend, wobei jede konkatemere Kopie des VSDL-Peptids durch eine N-polig gelegene Peptidspaltstelle und durch eine C-polig gelegene Peptidspaltstelle flankiert wird, wobei die Peptidspaltstellen ansonsten abwesend von dem VSDL-Peptid sind; und
Spalten des Fusionspolypeptids an der/den Peptidspaltungsstelle(n) mit einem Spaltungsmittel/mit Spaltungsmitteln, das/die an der/den Peptidspaltungsstelle(n) spaltet;
wobei der Schritt des Spaltens des Fusionspolypeptids das Peptid aus dem Fusionspolypeptid freigibt;
**dadurch gekennzeichnet, dass**:
das VSDL-Peptid aus einer natürlichen Peptidsequenz besteht, die einen C-poligen Argininrest umfasst;
die C-polig gelegene Peptidspaltungsstelle jeder konkatemeren Kopie des VSDL-Peptids aus dem C-poligen Argininrest besteht; und
der C-polige Argininrest jedes VSDL-Peptids außer des letzten VSDL-Peptids in dem Fusionspolypeptid zusätzlich als die N-polig gelegene Peptidspaltungsstelle eines folgenden VSDL-Peptids dient, wobei das VSDL-Peptid die Aminosäuresequenz nach SEQ ID NO: 2 umfasst oder daraus besteht.

2. Verfahren nach Anspruch 1, wobei die Peptidspaltungsstellen Trypsin-Spaltungsstellen sind und das Spaltungsmittel Trypsin oder ein Trypsin ähnliches Enzym ist.

3. Verfahren nach einem der Ansprüche 1 oder 2, ferner die folgenden Schritte umfassend:
Einstellen des pH-Wertes des gespalteten Fusionspolypeptids auf unter 5; und
Reinigen des Peptids von dem digerierten Fusionspolypeptid unter Verwendung wenigstens eines Anionenaustauschprozesses.

4. Verfahren nach einem der Ansprüche 1 bis 3, ferner das Sprüh- oder Gefriertrocknen des Peptids umfassend.

5. Verfahren nach einem der Ansprüche 1 bis 4, wobei das Fusionspolypeptid zwei bis zwanzig konkatemere Kopien des Peptids umfasst.

6. Verfahren nach einem der Ansprüche 1 bis 4, wobei das Fusionspolypeptid drei konkatemere Kopien des Peptids umfasst.

7. Verfahren nach einem der Ansprüche 1 bis 4, wobei das Fusionspolypeptid zehn konkatemere Kopien des Peptids umfasst.

8. Verfahren nach einem der Ansprüche 1 bis 7, wobei das Fusionspolypeptid einen führenden Fusionspartner mit einer C-poligen Peptidspaltungsstelle umfasst.

9. Verfahren nach Anspruch 8, wobei der führende Fusionspartner einen C-poligen Arginin- oder Lysinrest umfasst.

10. Expressionskonstrukt, eine Nukleotidsequenz umfassend, die für einen führenden Fusionspartner codiert, der wirkfusioniert mit wenigstens einer Nukleotidsequenz, die für zwei oder zwanzig konkatemere Kopien eines VSDL-Peptids codiert, ist, wobei der führende Fusionspartner eine C-polige Trypsinspaltungsstelle aufweist;
**dadurch gekennzeichnet, dass**:
das VSDL-Peptid aus einer natürlichen Peptidsequenz, die einen C-poligen Argininrest umfasst, besteht;
eine C-polig gelegene Peptidspaltungsstelle jeder konkatemeren Kopie des VSDL-Peptids aus dem C-poligen Argininrest besteht;
und der C-polige Argininrest jedes VSDL-Peptids außer des letzten VSDL-Peptids in dem Fusionspolypeptid zusätzlich als die N-polig gelegene Peptidspaltungsstelle eines folgenden VSDL-Peptids dient, wobei das VSDL-Peptid eine Aminosäuresequenz nach SEQ ID NO: 2 umfasst oder daraus besteht.

11. Wirtszelle zum rekombinanten Expremieren eines Peptids, wobei die Wirtszelle das Expressionskonstrukt nach Anspruch 10 umfasst.

12. Rekombinantes Fusionspolypeptid, einen führenden Fusionspartner umfassend, der mit zwei bis zwanzig konkatemeren Kopien eines VSDL-Peptids verbunden ist, wobei der führende Fusionspartner eine C-polige Trypsinspaltungsstelle aufweist;
**dadurch gekennzeichnet, dass**:
das VSDL-Peptid aus einer natürlichen Peptidsequenz, die einen C-poligen Argininrest umfasst, besteht;
eine C-polig gelegene Peptidspaltungsstelle jeder konkatemeren Kopie des VSDL-Peptids aus dem C-poligen Argininrest besteht;
und der C-polige Argininrest jedes VSDL-Peptids außer des letzten VSDL-Peptids in dem Fusionspolypeptid zusätzlich als die N-polig gelegene Peptidspaltungsstelle dient, wobei das VSDL-Peptid eine Aminosäuresequenz nach SEQ ID NO: 2 umfasst oder daraus besteht.

## Revendications

1. Procédé de production d'un peptide vasodilatateur (VSDL) recombinant, le procédé comprenant les étapes suivantes :
exprimer un polypeptide de fusion comprenant des copies concatémères du peptide VSDL dans lequel chaque copie concatémère du peptide VSDL est flanqué d'un site de clivage peptidique N-terminal et d'un site de clivage peptidique C-terminal, dans lequel lesdits sites de clivage peptidiques sont par ailleurs absents du peptide VSDL ; et
cliver le polypeptide de fusion au niveau du ou des sites de clivage peptidiques avec un ou des agents de clivage qui clivent au niveau du ou des sites de clivage peptidiques ;
dans lequel l'étape de clivage du polypeptide de fusion libère ledit peptide du polypeptide de fusion ;
**caractérisé en ce que** :
le peptide VSDL est constitué d'une séquence peptidique native comprenant un résidu d'arginine C-terminale ;
le site de clivage peptidique C-terminal de chaque copie concatémère du peptide VSDL est constitué du résidu d'arginine C-terminale ; et
le résidu d'arginine C-terminale de chaque peptide VSDL, à l'exception du dernier peptide VSDL dans le polypeptide de fusion, sert en outre de site de clivage peptidique N-terminal d'un peptide VSDL suivant, dans lequel le peptide VSDL comprend, ou est constitué de, la séquence d'acides aminés selon la SEQ ID NO : 2.

2. Procédé selon la revendication 1, dans lequel les sites de clivage peptidiques sont des sites de clivage de la trypsine, et l'agent de clivage est la trypsine ou une enzyme de type trypsine.

3. Procédé selon la revendication 1 ou 2, comprenant en outre les étapes suivantes :
ajuster le pH du polypeptide de fusion clivé en dessous de 5 ; et
purifier le peptide du polypeptide de fusion digéré en utilisant au moins une technique d'échange d'anions.

4. Procédé selon l'une quelconque des revendications 1 à 3, comprenant en outre le séchage par atomisation ou la lyophilisation du peptide.

5. Procédé selon l'une quelconque des revendications 1 à 4, dans lequel le polypeptide de fusion comprend de deux à vingt copies concatémères du peptide.

6. Procédé selon l'une quelconque des revendications 1 à 4, dans lequel le polypeptide de fusion comprend trois copies concatémères du peptide.

7. Procédé selon l'une quelconque des revendications 1 à 4, dans lequel le polypeptide de fusion comprend dix copies concatémères du peptide.

8. Procédé selon l'une quelconque des revendications 1 à 7, dans lequel le polypeptide de fusion comprend un partenaire de fusion leader ayant un site de clivage peptidique C-terminal.

9. Procédé selon la revendication 8, dans lequel le partenaire de fusion leader comprend une arginine C-terminale ou un résidu de lysine.

10. Construction d'expression comprenant une séquence nucléotidique codant pour un partenaire de fusion leader fusionné de manière opérationnelle avec au moins une séquence nucléotidique codant pour deux à vingt copies concatémères d'un peptide VSDL, dans laquelle le partenaire de fusion leader a un site de clivage de la trypsine C-terminale ; **caractérisé en ce que** :
le peptide VSDL est constitué d'une séquence peptidique native comprenant un résidu d'arginine C-terminale ;
un site de clivage peptidique C-terminal de chaque copie concatémère du peptide VSDL est constitué du résidu d'arginine C-terminale ;
et le résidu d'arginine C-terminale de chaque peptide VSDL, à l'exception du dernier peptide VSDL dans le polypeptide de fusion, sert en outre de site de clivage peptidique N-terminal d'un peptide VSDL suivant, dans laquelle le peptide VSDL comprend une, ou est constitué d'une, séquence d'acides aminés selon la SEQ ID NO : 2.

11. Cellule hôte pour exprimer de manière recombinante un peptide, la cellule hôte comprenant la construction d'expression selon la revendication 10.

12. Polypeptide de fusion recombinant comprenant un partenaire de fusion leader lié à deux à vingt copies concatémères d'un peptide VSDL, dans lequel le partenaire de fusion leader a un site de clivage de la trypsine C-terminale ;
**caractérisé en ce que** :
le peptide VSDL est constitué d'une séquence peptidique native comprenant un résidu d'arginine C-terminale ;
un site de clivage peptidique C-terminal de chaque copie concatémère du peptide VSDL est constitué du résidu d'arginine C-terminale ;
et le résidu d'arginine C-terminale de chaque peptide VSDL, à l'exception du dernier peptide VSDL dans le polypeptide de fusion, sert en outre de site de clivage peptidique N-terminal d'un peptide VSDL suivant, dans laquelle le peptide VSDL comprend une, ou est constitué d'une, séquence d'acides aminés selon la SEQ ID NO : 2.
